(19) Europäisches Patentamt
European Patent Office
Office européen des brevets

(11) EP 0 799 891 A1

(12) **EUROPEAN PATENT APPLICATION**

(43) Date of publication:
**08.10.1997 Bulletin 1997/41**

(21) Application number: **97302415.1**

(22) Date of filing: **27.03.1997**

(51) Int Cl.[6]: **C12N 15/13**, C07K 16/28, C12N 15/70, C12N 1/21, A61K 39/395

(84) Designated Contracting States:
**AT BE CH DE DK ES FI FR GB GR IE IT LI LU MC NL PT SE**

(30) Priority: **01.04.1996 JP 78570/96**

(83) **Declaration under Rule 28(4) EPC (expert solution)**

(71) Applicant: **SANKYO COMPANY LIMITED**
**Chuo-ku, Tokyo 103 (JP)**

(72) Inventors:
- **Serizawa, Nobufusa**
  **Shinagawa-ku, Tokyo 140 (JP)**
- **Ichikawa, Kimihisa**
  **Shinagawa-ku, Tokyo 140 (JP)**
- **Nakahara Kaori**
  **Shinagawa-ku, Tokyo 140 (JP)**
- **Yonehara, Shin**
  **Kyoto-shi, Kyoto-fu (JP)**

(74) Representative: **Gibson, Christian John Robert**
**MARKS & CLERK,**
**57/60 Lincoln's Inn Fields**
**London WC2A 3LS (GB)**

(54) **Anti-Fas recombinant antibodies and DNA therefor**

(57) A recombinant protein comprises at least one variable region corresponding to an immunoglobulin variable region specific for human Fas and has substantially no more immunogenicity in a human than a human antibody, and is useful in the treatment of autoimmune diseases, especially rheumatism.

EP 0 799 891 A1

## Description

The present invention relates to recombinant antibodies directed against Fas, especially human Fas, and to DNA encoding such antibodies. The present invention further relates to pharmaceutical preparations containing such antibodies and being for the treatment of autoimmune diseases including rheumatoid arthritis, the preparations optionally further containing a cell growth inhibitor. The present invention also relates to anti-Fas complementarity determining regions (CDR's) and their use in the creation of such antibodies, and to DNA encoding such CDR's.

The physiological death of cells in a living organism in the natural course of events is known as apoptosis, and is distinguished from the pathological death of cells, i.e. necrosis [*c.f.* Kerr *et al.,* (1972), Br. J. Cancer, *26*, 239 *et seq.*]. Apoptosis is an example of programmed cell death, which is where certain cells are programmed in advance to die in a living organism in the natural course of events, such as when the cell in question has performed a pre-determined function. Apoptosis is characterised by such morphological changes as curved cell surface, condensed nuclear chromatin and fragmented chromosomal DNA, amongst others.

Apoptosis has an important rôle to play in disposing of cells that recognise autoantigen during the process of T and B lymphocyte differentiation. Onset of so-called autoimmune diseases is generally brought on by the appearance of auto-reactive lymphocytes resulting from the failure of apoptosis during lymphocyte differentiation [*c.f.* Keiichi Nakayama *et al.,* (1995), Mebio *12(10),* 79-86].

Fas is a cell membrane molecule involved in apoptosis of immunocompetent cells [*c.f.* Yonehara, S., *et al.* (1989), J. Exp. Med. *169*, 1747 *et seq.;* Trauth, B. C., *et al.* (1989), Science *24*, 301 *et seq.;* and Itoh, N., *et al., infra*]. Murine monoclonal antibodies have been generated against the human Fas antigen [*c.f.* Itoh, N., *et al.* (1991), Cell *66*, 233-243 and Yonehara, *supra,* who discloses a monoclonal antibody designated CH11 specific for human Fas]. These anti-human Fas antibodies have apoptosis-inducing cytotoxic activity, and have been proposed as potential therapeutic agents for autoimmune diseases, such as AIDS, as well as tumours [*c.f.* Japanese Unexamined Patent Publication No. Hei 2-237935 and Japanese Unexamined PCT Publication No. Hei 5-503281]. Ogasawara *et al.* [Nature (1993), *364*, 806-809] report that murine anti-Fas antibodies rapidly kill wild-type mice, evidently by massive apoptosis in the liver.

Monoclonal antibodies against human Fas are also known from the following publications: J. Exp. Med. (1989), *169*, 1747-1756; WO 91/10448; Science (1989), *245,* 301-305; Cell (1991), *66*, 233-243; J. Imm. (1992), *149,* 3166-3173; and Internat. Immun. (1994), *6*, (12) 1849-1856.

Rheumatism, especially rheumatoid arthritis, is believed to result from the proliferation of synoviocytes, accompanied by a variety of immunological abnormalities. The proliferation of synoviocytes is typically accompanied by inflammatory cellular infiltration and erosion of bone. Tissue erosion around the affected joint in chronic rheumatoid arthritis is apparently caused by abnormal production of cytokines from inflammatory synoviocytes. Examination of joints in patients with rheumatism reveals abnormal proliferation of synoviocytes, hyperplasia of synovial villi, multi-layered synoviocytes, etc. [*c.f.* Daniel J. McCarty (1985), in "Arthritis and allied conditions, A textbook of rheumatology" 10th Edition, Lea & Febiger]. Medication for rheumatism currently predominantly comprises anti-inflammatory drugs such as steroids and immunomodulators. If it were possible to inhibit abnormal proliferation of synoviocytes, then any such agent should be useful in the therapy of rheumatism.

Synoviocytes in rheumatism do not proliferate in an unlimited manner [*c.f.* Daniel J. McCarty (1985), in "Arthritis and allied conditions, A textbook of rheumatology" 10th Edition, Lea & Febiger], and it has been demonstrated that apoptosis occurs in the synoviocytes of patients with rheumatism Fas antigen is expressed on the membrane of synoviocytes and Nakajima *et al.* [Nakajima, T., *et al.* (1995), Arthritis Rheum. *38*, 485-491] and Aono *et al.* [Abstracts of the 38th Meeting of Japan Rheumatism Society (1994), p. 487, and articles of 1994 Meeting of Japan Cancer Society, (1994), p. 388] investigated whether cytotoxic anti-human Fas antibodies could induce apoptosis in abnormally proliferated synoviocytes from patients with rheumatism. They were able to induce high levels of apoptosis in abnormally proliferated synoviocytes from patients with rheumatism, compared with a control comprising synoviocytes from patients with diseases other than rheumatism.

Thus, anti-human Fas antibody is able to selectively induce apoptosis not only in lymphocytes but also in abnormally proliferated synoviocytes, so that anti-human Fas antibody should be useful as an antirheumatic agent.

However, administration of murine monoclonal antibodies to humans as a pharmaceutical preparation intended for chronic administration is highly likely to cause a variety of problems, owing to the antigenicity arising from the murine origin. Anaphylactic shock is likely to occur, and repeated dosage may reduce efficacy or hasten reduction in blood levels etc., which effects are undesirable if the antibody is to be used in clinical practices.

Efforts to overcome the problems associated with murine monoclonal antibodies have involved the use of recombinant DNA technology in order to obtain a chimaeric antibody wherein the constant region of the mouse antibody has been replaced with the constant region of the corresponding human antibody [*c.f.* Morrison, S. L., *et al.* (1984), Proc. Natl. Acad. Sci. USA, *81*, 6851-6855]. In an alternative process of humanisation of the murine antibody, the murine CDR present in the variable region of mouse antibody [*c.f.* Jones, P. T., *et al.* (1986), Nature, *321*, 522-525] has been inserted into human antibody in an essentially equivalent process. In order to prepare such a genetically engineered antibody, it is first necessary to clone DNA encoding both the H and the L chain subunits which constitute the mouse antibody. These chains specifically recognise the target antigen, so that elucidation of their sequences reveals the primary structure of the variable region containing the CDR, and this can then be used in the preparation of a humanised antibody.

To date, there has been no disclosure of any part of the CDR of a cytotoxic mouse anti-human Fas monoclonal antibody, or of a DNA sequence therefor.

Thus, in a first aspect, the present invention provides a recombinant protein, the protein comprising at least one region corresponding to an immunoglobulin variable region, wherein the at least one region enables the protein to recognise and specifically bind to an antigen, the antigen being human Fas, and wherein the protein has substantially no more immunogenicity in a human patient than a human antibody.

Thus, it will be appreciated that the present invention allows the construction of humanised antibodies specific for human Fas from variable regions recognising human Fas.

As used herein, the term "recombinant" relates to any substance which has been obtained by genetic engineering, insofar as the substance in question is either modified from the original substance, or expressed in a different manner or in a different system than the original.

The term "protein" relates generally to any sequence of amino acid residues linked by peptide bridges, and may encompass oligopeptides as well as polypeptides and multimeric peptide or polypeptide assemblies.

The region corresponding to an immunoglobulin variable region has a structure characteristic of an immunoglobulin variable region, and may correspond to the variable region of either the H chain or the L chain of an immunoglobulin. In the preferred embodiments, the proteins of the present invention have at least two such regions, one corresponding to an H chain variable region, and one corresponding to an L chain variable region, both of which are specific for human Fas.

The proteins of the present invention have substantially no more immunogenicity in a human patient than a human antibody, owing to the fact that the part of the antibody corresponding to a heterologous constant region is not present. Thus, the proteins of the present invention may have a variable region or regions originating from a murine monoclonal antibody, but the murine constant region has been eliminated. As a result, the proteins of the invention only have the same level of immunogenicity as, for example, a human antibody from an alternative source to the patient, owing to the fact that murine variable regions are sufficiently similar to those of humans, that any raised level of immunogenicity can only come from any other amino acid sequence of which the variable region(s) is a part.

The variable regions of the present invention may derive from any source from which it is possible to generate antibodies against human Fas. Although this is most conveniently the mouse, other sources, such as rats and rabbits, are also possible, although these and higher animals tend to be less convenient.

The variable regions of the present invention may be present in the protein in any suitable configuration. At its simplest, the protein may consist essentially of only one variable region, although the efficacy of such proteins tends to be greatly reduced. In part, this is due to lack of stabilisation of the tertiary structure, although binding may still be observed.

At a higher level, the protein may consist essentially of one H chain variable region and one L chain variable region together in association. Such association may be by direct linkage, or may be in the form of a flexible peptide linkage involving one or more extra amino acid residues. Such constructs have the advantage that they do not have any extra peptide sequence to give rise to potential immunogenicity.

Although it is possible for proteins of the present invention to only have one variable region, it is preferred that they have at least two. Further, it is preferred that the two are derived from one particular antibody, such as CH11, one each from the H and L chains, thereby to more closely emulate a natural antibody. In this respect, it is desirable that the variable sequences are integrally bound with human constant regions, especially the appropriate constant regions from the H and L chains.

In the above embodiment, it is preferred that the two variable regions are each respectively a part of a larger polypeptide and the two polypeptides are able to associate to form an antibody heterodimer.

The nature of the antibody is not crucial to the present invention, and may be of any appropriate type, where the protein of the invention actually corresponds to an antibody type. For example, the type may be IgG, IgM or IgE, and the type may be entirely dependent on administration path, for example. Although CH11 is an IgM molecule, the variable regions can just as well be put into an IgG construct. In fact, we have discovered that, if an IgM type construct is used, but without the J chain, which forms a pentameric antibody structure with 5 heavy and light chain pairs, then apoptotic activity is increased by five-fold or even more.

It will be appreciated that the present invention further provides DNA and RNA encoding any of the above identified proteins, especially DNA. the DNA may simply encode the protein, or may encode a particular polypeptide, if the protein is heterodimeric, for example.

It will also be appreciated that the DNA may be in any suitable form, so that it may be incorporated into a vector, suitably an expression vector. It may also be associated with any other suitable sequences, such as leader sequences or sequences for the expression of the encoded protein in the form of a fusion protein, for example.

The present invention further envisages a host cell transformed with a vector as defined above, and a system for the expression of a protein of the invention comprising such a host cell transformed with one or more expression vectors containing the above DNA. The protein of the invention may be obtained from such systems, after cultivation of the system, by standard techniques.

Certain preferred aspects and embodiments of the present invention now follow:

A genetically engineered immunoglobulin protein which specifically binds to human Fas, wherein the immunoglobulin protein consists essentially of an H chain subunit and an L chain subunit, the H chain subunit comprising an amino acid sequence represented by the following general formula (I):

$$-FRH_1-CDRH_1-FRH_2-CDRH_2-FRH_3-CDRH_3-FRH_4- \quad (I)$$

wherein $FRH_1$ represents an amino acid sequence comprising 13 to 30 amino acid residues, $CDRH_1$ represents the amino acid sequence of SEQ ID NO. 1, $FRH_2$ represents an amino acid sequence comprising 14 amino acid residues, $CDRH_2$ represents the amino acid sequence of SEQ ID NO. 2, $FRH_3$ represents an amino acid sequence comprising 32 amino acid residues, $CDRH_3$ represents the amino acid sequence of SEQ ID NO. 3, and $FRH_4$ represents an amino acid sequence comprising 11 amino acid residues, each of the amino acid sequences being linked to the next *via* a peptide bond;

the L chain subunit comprising an amino acid sequence represented by the following general formula (II):

$$-FRL_1-CDRL_1-FRL_2-CDRL_2-FRL_3-CDRL_3-FRL_4- \quad (II)$$

wherein $FRL_1$ represents an amino acid sequence comprising 23 amino acid residues, $CDRL_1$ represents the amino acid sequence of SEQ ID NO. 4, $FRL_2$ represents an amino acid sequence comprising 15 amino acid residues, $CDRL_2$ represents the amino acid sequence of SEQ ID NO. 5, $FRL_3$ represents an amino acid sequence comprising 32 amino acid residues, $CDRL_3$ represents the amino acid sequence of SEQ ID NO. 6, and $FRL_4$ represents an amino acid sequence comprising 10 amino acid residues, each of the amino acid sequences being linked to the next *via* a peptide bond.

The above protein is preferred, when the H chain subunit comprises the amino acid sequence represented by amino acids No. 1 to 116 in SEQ ID NO. 8, and/or wherein the L chain subunit comprises the amino acid sequence represented by amino acids No. 1 to 112 in SEQ ID NO. 10.

The above protein is more preferred when the H chain subunit comprises the amino acid sequence represented by amino acids No. 1 to 571 in SEQ ID NO. 8, and/or the L chain subunit comprises the amino acid sequence represented by amino acids No. 1 to 219 in SEQ ID NO. 10.

The invention further provides DNA encoding an H chain subunit comprising a peptide of formula (I) as defined above. We prefer such DNA when it comprises the nucleotide sequence represented by nucleotides No. 58 to 405 in SEQ ID NO. 7.

Also preferred is DNA which hybridises with such DNA, the sense strand encoding an immunoglobulin H chain subunit capable of specifically binding human Fas together with an L chain subunit comprising an amino acid sequence of general formula (II). It is preferred that such DNA is able to hybridise at 60 to 70°C and in 6 x SSC.

DNA coding for an L chain subunit comprising an amino acid sequence represented by general formula (II) is also preferred, especially where it comprises the nucleotide sequence represented by nucleotides No. 58 to 393 in SEQ ID NO. 9. DNA which hybridises such DNA and whose corresponding sense strand encodes an immunoglobulin L chain subunit which specifically binds to human Fas antigen together with an H chain subunit as defined above which comprises an amino acid sequence of general formula (I) is also preferred, especially where hybridisation is at 60 to 70°C in 6 x SSC.

Further preferred is a recombinant DNA vector containing any of the DNA described above, particularly recombinant DNA vectors pCR3-H123 and pCR3-L103, cells transformed with such vectors and especially *E. coli* pCR3-H123 (FERM BP-5247) and *E. coli* pCR3-L103 (FERM BP-5248).

A preferred method for producing an immunoglobulin protein which specifically recognises human Fas antigen comprises:

culturing a cell transformed by a DNA vector described above under conditions which enable expression of DNA encoding the immunoglobulin H chain or L chain subunit contained in the vector, and

recovering the immunoglobulin protein from the culture.

The present invention further envisages the use of CDR's, whether or not linked by framework regions (FR's) as identified above, and DNA encoding such, in the construction of humanised antibodies and proteins in general, which selectively bind human Fas.

Essentially, we have now successfully cloned the genes encoding each of the H, L and J chains of an anti-human Fas, IgM monoclonal antibody. A cDNA library prepared from a hybridoma producing the antibody was used, and it was possible to elucidate the entire nucleotide sequence of each gene and their corresponding amino acid sequences. From these, it was possible to obtain the CDR sequences of the H and L chains. Expression vectors comprising the H, L and J chains were constructed, and it was found that cytotoxic proteins having similar activity to anti-human Fas antibody were obtainable from the supernatant of a culture obtained by co-transfecting cultured animal cells with these vectors.

The DNA of the present invention may be obtained by first preparing poly(A)$^+$ RNA from mouse hybridoma cells producing anti-human Fas monoclonal antibody, such as CH11. The poly(A)$^+$ RNA may then be converted to cDNA using a reverse transcriptase, and purifying the cDNA encoding the H, L and, optionally, the J chains of the antibody. Yonehara *et al.* [(1989), J. Exp. Med. *169*, 1747 *et seq.*] obtained an anti-human Fas monoclonal antibody, which was designated CH11, by fusion of mouse myeloma cells with mouse lymphocytes after the mice had been immunised with the Fas-expressing human diploid fibroblast cell-line FS-7. The CH11 antibody is commercially available from Medical Biological Laboratory Co. Ltd. of Japan.

Poly(A)$^+$ RNA may be obtained either by first preparing total RNA and then purifying poly(A)$^+$ RNA from the total RNA using, for example an affinity column packed with oligo(dT) cellulose, oligo(dT) latex beads etc., or it may be obtained by directly purifying poly(A)$^+$ RNA from cell lysates using such affinity materials as described above. Total RNA may be prepared, for example, by such methods as: alkaline sucrose density gradient ultracentrifugation [*c.f.* Dougherty, W. G. and Hiebert, E., (1980), Virology, *101*, 466-474]; the guanidine thiocyanate-phenol method; the guanidine thiocyanate-trifluorocaesium method; and the phenol-SDS method. The preferred method, however, employs guanidine thiocyanate and caesium chloride [*c.f.* Chirgwin, J. M., *et al.* (1979), Biochemistry, *18*, 5294-5299].

The single stranded (ss) cDNA obtained by the use of reverse transcriptase, as described above, can then be converted to double stranded (ds) cDNA. Suitable methods for obtaining the ds cDNA include the S1 nuclease method [*c.f.* Efstratiadis, A., *et al.* (1976), Cell, *7*,279-288] and the Gubler-Hoffman method [*c.f* Gubler, U. and Hoffman, B. J. (1983), Gene, *25*, 263-269]. However, we prefer to employ the Okayama-Berg method [*c.f.* Okayama, H. and Berg, P. (1982), Mol. Cell. Biol. *2*, 161-170].

The ds cDNA obtained above may then be integrated into a cloning vector and the resulting recombinant vector can then be used to transform a suitable microorganism, such as *E. coli.* The transformant can be selected using a standard method, such as by selecting for tetracycline resistance or ampicillin resistance encoded by the recombinant vector. If *E. coli* is used, then transformation may be effected by the Hanahan method [*c.f.* Hanahan, D. (1983), J. Mol. Biol., *166*, 557-580]. Alternatively, the recombinant vector may be introduced into competent cells prepared by co-exposure to calcium chloride and either magnesium chloride or rubidium chloride. If a plasmid is used as a vector, then it is highly desirable that the plasmid harbours a drug-resistant gene, such as mentioned above, in order to facilitate selection. Brute force selection is possible, but not preferred. Although plasmids have been discussed, it will be appreciated that other cloning vehicles, such as lambda phages, may be used.

Methods for selecting transformants having the desired DNA include the following:

(1) Screening using a synthetic oligonucleotide probe

If all or part of the amino acid sequence of the desired protein has been elucidated, then a short contiguous sequence, which is also representative of the desired protein, may be used to construct an oligonucleotide probe. The probe encodes the amino acid sequence but, owing to the degeneracy of the genetic code, there may be a large number of probes that can be prepared. Thus, an amino acid sequence will normally be selected which can only be encoded by a limited number of oligonucleotides. The number of oligonucleotides which it is necessary to produce can be further reduced by the substitution of inosine where any of the four normal bases can be used. The probe is then suitably labelled, such as with $^{32}P$, $^{35}S$ or biotin, and is then hybridised with denatured, transformed DNA from the transformant which has been immobilised on a nitrocellulose filter. Positive strains show up by detection of the label on the probe.

(2) Polymerase Chain Reaction

If all or part of the amino acid sequence of the desired protein has been elucidated, then sense and antisense oligonucleotide primers corresponding to separate non-contiguous parts of the amino acid sequence can be synthesised, such as by the methods described in (1) above. These primers can then be used in the polymerase chain reaction technique [*c.f.* Saiki, R. K., *et al.* (1988), Science, *239*, 487-491] using the template DNA. The template DNA used herein may be cDNA synthesised by a reverse transcriptase reaction using mRNA obtained from a hybridoma producing anti-human Fas antibody, such as that which expresses CH11. The DNA fragment thus synthesised may either be directly integrated into a plasmid vector, such as by using a commercial kit, or may be labelled with, for example, $^{32}P$, $^{35}S$ or biotin, and then used as a probe for colony hybridisation or plaque hybridisation to obtain the desired clone.

Monoclonal antibody CH11 is an immunoglobulin M ("IgM") molecule, a complex comprising five subunits each of the H (μ chain) and L chains, and one J chain. Thus, in order to elucidate partial amino acid sequences for the subunits, the subunits must be separated, and this can be done using any suitable technique, such as electrophoresis, column chromatography, etc. well known to those skilled in the art. Once the subunits have been separated, they can be sequenced, such as by the use of an automatic protein sequencer (for example, PPSQ-10 of Shimadzu), in order to determine the amino acid sequence of at least the N-terminal of each subunit. Oligonucleotides/primers can then be produced using this knowledge.

Harvesting of DNA encoding each subunit of anti-human Fas monoclonal antibody from the appropriate transformants obtained above may be performed by well known techniques, such as those described by Maniatis, T., *et al.* [in "Molecular Cloning A Laboratory Manual" Cold Spring Harbor Laboratory, NY, (1982)]. For example, the region of DNA coding for the desired subunit may be excised from plasmid DNA after separating the fraction corresponding to the vector DNA from a transformant which has been determined to possess the necessary plasmid.

*E. coli* DH5α has been transformed with plasmids containing DNA encoding the heavy, light and J chains of CH11, prepared as described above, and the resulting three transformants (designated *E. coli* pCR3-H123, *E. coli* pCR3-L103 and *E. coli* pCR3-J1123, respectively) have been deposited in accordance with the terms of the Budapest Treaty on the Deposition of Microorganisms at the Research Institute of Life Science Technology of the Agency of Industrial Science and Technology on February 28, 1996, and have been allocated deposit Nos. FERM BP-5427, FERM BP-5428 and FERM BP-5429, respectively. *E. coli* DH5α containing these plasmids may be cultivated in a directly comparable manner to *E. coli* DH5α not possessing these plasmids. All deposited strains may be selected by their resistance to ampicillin. The DNA of the present invention, therefore, may be obtained using these deposits. This can be done, for example, either by cultivating the deposits and isolating the plasmids, or by using the polymerase chain reaction (PCR) using the plasmids as templates.

Wherever appropriate, DNA sequences may be sequenced in accordance by various well known methods in the art including, for example, the Maxam-Gilbert chemical modification technique [*c.f.* Maxam, A. M. and Gilbert, W. (1980) in "Methods in Enzymology" *65*, 499-276] and the dideoxy chain termination method using M13 phage [*c.f.* Messing, J. and Vieira, J. (1982), Gene, *19*, 269-276]. In recent years, a further method for sequencing DNA has gained wide acceptance, and involves the use of a fluorogenic dye in place of the conventional radioisotope in the dideoxy method. The whole process is computerised, including the reading of the nucleotide sequence after electrophoresis. Suitable machinery for the process is, for example, the Perkin-Elmer Sequence robot "CATALYST 800" and the Perkin-Elmer model 373A DNA Sequencer. The use of this technique renders the determination of DNA nucleotide sequences both efficient and safe.

Accordingly, from the thus determined nucleotide sequences of the DNA encoding the H and L chains of CH11, in conjunction with the sequence data for the N-termini of the H and L chains, it was possible to determine the entire amino acid sequence of the H and L chains of CH11.

Framework regions (FR's) are present in the variable regions of the H and L chains of immunoglobulins and serve to flank the variable region and to separate the CDR's. Accordingly, there is one more FR compared to the number of CDR's in each variable region. The framework regions also contribute to the three-dimensional structure of the variable region, thereby assisting in antigenic binding by stabilising the tertiary structure of the region. By way of illustration of the terminology applied to each FR and CDR in the variable region, $FRH_1$ is the framework region at the N-terminal end of the variable region of the H chain. Working towards the C-terminal end, there is, respectively, $CDRH_1$, $FRH_2$, $CDRH_2$, $FRH_3$, $CDRH_3$ and, finally, $FRH_4$. A similar nomenclature applies to the L chain, so that the first framework region is designated $FRL_1$, etc.

Both immunoglobulin H and L chains comprise a variable region and a constant region. The variable region is composed of CDR's and, as described above, both the H chain and the L chain have three CDR's interconnected and flanked by four framework regions. The amino acid sequences of the constant regions are the same, irrespective of the antigen recognised, and this is true separately for the H and L chains, provided that the immunoglobulin class is the same. By contrast, the amino acid sequence of the variable region, particularly the CDR's, is peculiar to each antibody. However, according to a comparative study on amino acid sequences of a number of antibodies, both the location of the CDR's and the length of the framework sequences connecting them are virtually constant among antibody subunits of the same subgroup [Kabat, E. A., *et al.* (1991), in "Sequence of Proteins of Immunological Interest Vol. II": U.S. Department of Health and Human Services]. With this knowledge, therefore, it was possible to determine the location of the CDR's, framework regions and the constant region of each of the H and L chains of CH11 by comparison of the amino acid sequences we had determined with the known amino acid sequences.

It will be appreciated that the framework regions are important for separating the CDR's and help in the steric determination of structure of the variable regions, but that, provided that these requirements are met, then the actual size and sequence of each framework region is not critical to the present invention, given that the FR's are not responsible for binding the antigen. Accordingly, any suitable amino acid sequence can be used for each FR. However, it has been observed that framework regions are commonly conserved in nature and, for this reason, we generally prefer to retain regions which are either identical with or which correspond closely to those found in CH11 (Yonehara *et al., supra).*

Although the chain lengths of the framework region are generally substantially conserved, especially within immunoglobulin sub-types, it has been established that $FRH_1$, located at the N-terminal of the H chain may sometimes be shorter than expected. Thus, instead of 30 amino acids, $FRH_1$ may be as short as 13 amino acids, in mouse IgM, and a minimum length of 19 amino acids for a $\mu$ 2a subtype for CH11 has been established [*c.f.* Kabat *et al.* ibid.]. Accordingly, the chain length of the framework region at the N-terminal of the H chain may be anywhere between 13 and 30 amino acids. This range is preferably from 19 to 30 amino acids and is most preferably 30 amino acids.

In general, it will be appreciated that the framework regions at either end of the variable region ($FRH_1$ and $FRH_4$ in the H chain) are of less importance than the interstitial framework regions actually connecting the CDR's ($FRH_2$ and $FRH_3$ in the H chain). Accordingly, the flanking framework regions may vary by up to about 60% in length from the norm for any given antibody type, while the interstitial framework regions can vary in length to only a limited extent, which is typically only about one amino acid. In some cases, any variation in length may result in substantially reduced efficiency, and the skilled person in the art will easily be able to establish the requisite parameters for any given variable region construct.

If it is desired to construct an altered sequence in which any one or more amino acids are deleted in the amino acid sequence, then the cassette mutation method may be used, for example [*c.f.* Toshimitsu Kishimoto, "Shin-Seikagaku Jikken Kouza (New Biochemistry Experiment Series) II: Nucleic Acid III, Recombinant DNA Technology", pp. 242-251].

Any appropriate amino acid sequences may be bound to the C-terminal end of either variable region, provided that they do not adversely affect the antigen-binding ability of the antibody, unless this is for a reason, such as to form a precursor molecule, for example in the form of a leader sequence. In general, where there is a C-terminal sequence, then we prefer that it corresponds to a human constant region from the appropriate H or L chain.

DNA of the present invention may be prepared by any suitable method, such as by chemical synthesis using conventional methods, such as the phosphate-triester method [*c.f.* Hunkapiller, M., *et al.* (1984), Nature, *310*, 105-111; and Grantham, R., *et al.* (1981), Nucleic Acids Res. *9*, r43-r74]. The various codons for amino acids are well known, and their selection may be totally arbitrary, although it is generally preferred to make the selection based on the frequency of usage of the codons for the selected host. If a technique other than direct chemical synthesis is employed, then it may be desired to partially modify the codons of the sequence obtained, and this can be effected, for example, by site specific mutagenesis [*c.f.* Mark, D. F., *et al.* (1984), Proc. Natl. Acad. Sci. USA, *81*, 5662-5666] using, as primers, synthetic oligonucleotides incorporating the desired modification.

In the present invention, where it is specified that DNA can hybridise with other DNA, then this may be established by techniques well known in the art. For example, to establish whether a certain sequence hybridises with any other given sequence, then one or other sequence may be labelled with, for example, [$\alpha$-$^{32}$P] dCTP, using the random primer method [*c.f.* Feinberg A. P. and Vogelstein, B. (1983), Anal. Biochem., *132*, 6-13] or the nick translation method [Maniatis, T., *et al.* (1982), in "Molecular Cloning A Laboratory Manual" Cold Spring Harbor Laboratory, NY], for example. One DNA strand used in a hybridisation test is suitably adsorbed to, for example, a nitrocellulose membrane or a nylon membrane, and immobilised thereon by heating or UV irradiation using conventional methods. In one specific embodiment, the membrane may then be immersed in a prehybridisation solution containing 6 x SSC, 5% (v/v) Denhardt's solution and 0.1% (w/v) sodium dodecyl sulphate (SDS), and incubated at 55°C for over 4 hours. The other, labelled strand is then added to the prehybridisation solution to a final specific activity of 1 x $10^6$ cpm/ml and incubated overnight at 60°C. Thereafter, the membrane is washed in 6 x SSC at room temperature five times each for 5 minutes. After further washing at 57°C for 20 minutes, the membrane is then subjected to autoradiography to determine whether hybridisation has taken place. Thus, DNA hybridising with DNA of the present invention can be prepared and isolated [Maniatis, T., *et al.* (1982) in "Molecular Cloning A Laboratory Manual" Cold Spring Harbor Laboratory, NY].

Integration of DNA of the present invention thus obtained into an expression vector allows transformation of prokaryotic or eukaryotic host cells. Such expression vectors will typically contain suitable promoters, replication sites and sequences involved in gene expression, allowing the DNA to be expressed in the host cell.

Suitable prokaryotic host cells include, for example, *E. coli (Escherichia coli)* and *Bacillus subtilis.* In order to express the gene of interest in such host cells, these host cells may be transformed with a plasmid vector containing a replicon derived from a species compatible with the host, typically having an origin of replication and a promoter sequence, such as lac UV5. These vectors preferably have sequences capable of conferring a selection phenotype on the transformed cell.

A suitable strain *of E. coli* is strain JM109 derived from *E. coli* K12. Suitable vectors include pBR322 and the pUC series plasmids. Suitable promoters include the lactose promoter (lac) and the tryptophan lactose promoter (trc). In general, it will be appreciated that the present invention is not limited to the use of such hosts, vectors, promoters, etc., as exemplified herein and that any suitable systems may be used, as desired.

A suitable preferred strain of *Bacillus subtilis* is strain 207-25, and a preferred vector is pTUB228 [*c.f.* Ohmura, K., *et al.* (1984), J. Biochem., 95, 87-93]. A suitable promoter is the regulatory sequence of the *Bacillus subtilis* α-amylase gene. If desired, the DNA sequence encoding the signal peptide sequence of α-amylase may be linked to the DNA of the present invention to enable extracellular secretion.

Suitable eukaryotic cell hosts include those from vertebrates, yeasts, etc. Suitable vertebrate cells include, for example, the monkey cell line COS [*c.f.* Gluzman, Y. (1981), Cell, *23*, 175-182]. Suitable yeasts include *Saccharomyces cerevisiae* and *Schizosaccharomyces pombe.*

In general, the requirements for suitable expression vectors for vertebrate cells are that they comprise: a promoter usually upstream of the gene to be expressed; an RNA splicing site; a polyadenylation site; and a transcription termination sequence, etc. As desired, they may additionally contain, as needed, an origin of replication. A suitable plasmid is pSV2dhfr containing the SV40 early promoter [*c.f.* Subramani, S., *et al.* (1981), Mol. Cell. Biol., *1*, 854-884].

Suitable eukaryotic microorganisms are the yeasts, such as *S. cerevisiae,* and suitable expression vectors for yeasts include pAH301, pAH82 and YEp51. Suitable vectors contain, for example, the promoter of the alcohol dehydrogenase gene [*c.f.* Bennetzen, J. L. and Hall, B. D. (1982), J. Biol. Chem., *257*, 3018-3025] or of the carboxypeptidase Y GAL10 promoter [*c.f.* Ichikawa, K., *et al.* (1993), Biosci. Biotech. Biochem., *57*, 1686-1690]. If desired, the DNA sequence encoding the signal peptide sequence of carboxypeptidase Y may be linked to the DNA to be expressed in order to enable extracellular secretion.

In the case of COS cells being used as hosts, suitable vectors comprise the SV40 replication origin, enabling autonomous growth, a transcription promoter, a transcription termination signal and an RNA splicing site. The expression vectors can be used to transform the cells by any suitable method, such as the DEAE-dextran method [*c.f.* Luthman, H, and Magnusson, G. (1983), Nucleic Acids Res., *11*, 1295-1308], the phosphate calcium-DNA co-precipitation method [*c.f.* Graham, F. L. and van der Eb, A. J. (1973), Virology, *52*, 456-457] and the electric pulse electroporation method [*c.f.* Neumann, E., *et al.* (1982), EMBO J., 1, 841-845]. In a preferred embodiment COS cells are co-transfected with two separate expression vectors - one containing DNA encoding a protein comprising the variable region of the H chain of CH11 and one containing DNA encoding a protein comprising the variable region of the L chain of CH11, these vectors being expressed simultaneously to generate a recombinant anti-human Fas antibody.

Transformants of the present invention may be cultured using conventional methods, the desired proteins being expressed either intra- or extra- cellularly. Suitable culture media include various commonly used media, and will generally be selected according to the host chosen. For example, suitable media for COS cells include RPMI-1640 and Dulbecco's Modified Eagle Minimum Essential medium which can be supplemented with, as desired, foetal bovine serum (FBS). The culture temperature may be any suitable temperature which does not markedly depress the protein synthesis capability of the cell, and is preferably in the range of 32 to 42°C, most preferably 37°C, especially for mammalian cells. If desired, culture may be effected in an atmosphere containing 1 to 10% (v/v) carbon dioxide.

The protein expressed by the transformants of the present invention may be isolated and purified by various well known methods of separation according whether the protein is expressed intra- or extra- cellularly and depending on such considerations as the physical and chemical properties of the protein. Suitable specific methods of separation include: treatment with commonly used precipitating agents for protein; various methods of chromatography such as ultrafiltration, molecular sieve chromatography (gel filtration), adsorption chromatography, ion exchange chromatography, affinity chromatography and high performance liquid chromatography (HPLC); dialysis; and combinations thereof.

By the use of such methods as described above, the desired protein can be readily obtained in high yields and high purity. The antibody produced in the preferred embodiment may lack the J chain if there is no extra plasmid encoding the J chain. In such an instance, we have established that the resulting H and L chain heterodimers have a cytotoxicity about five times greater than that of CH11.

The specific binding activity of proteins of the present invention for Fas antigen may be determined, for example, by enzyme-linked immunosorbent assay (ELISA). In one specific embodiment of ELISA, Fas antigen is immobilised on the bottom of wells of a 96-well plate and test protein is introduced to the wells, whereafter the cells are washed to remove unbound protein. After washing, the wells are exposed to an enzyme-labelled antibody specific for the protein, such as the constant region thereof. The cells are then washed again, and any label remaining in the well is detected. cDNA encoding for human Fas antigen has previously been disclosed and methods for introducing the cDNA into animal cells for expression thereof are also known [*c.f.* Itoh, N., *et al.* (1991), Cell, *66*, 233-243]. Antigen for use in the above ELISA method can be obtained from the culture supernatant of cells which have been transformed with an expression vector containing the gene encoding a fusion protein comprising the extracellular region of the human Fas antigen and the extracellular region of mouse interleukin 3 receptor, as disclosed in Itoh (*supra*).

Cytotoxicity of the proteins of the present invention can be established, for example, by culturing cells expressing Fas {for example, human lymphocyte cell line HPB-ALL [*c.f.* Morikawa, S., *et al.* (1978), Int. J. Cancer, *21*, 166-170] and Jurkat (American Type Culture No. TIB-152)} in medium into which the test sample has been or will be added, and determining the survival ratio by MTT Assay [*c.f.* Green, L. M., *et al.* (1984), J. Immunological Methods, *70*, 257-268].

Using the DNA of the present invention, chimaeric mouse antibody wherein the constant regions have been replaced with those of human immunoglobulin [*c.f.* Morrison, S. L., *et al.* (1984), Proc. Natl. Acad. Sci. USA, *81*, 6851-6855] can be constructed.

Likewise, it is possible to construct an Fv fraction composed essentially only of the variable regions of the H and L chains, this being a single chain Fv (scFv) [*c.f.* Huston, J. S., *et al.* (1988), Proc. Natl. Acad. Sci. USA, *85*, 5879 *et seq.*] wherein the H and L chains are linked by a flexible peptide.

Thus, in accordance with the present invention, anti-Fas antibody having a reduced immunogenicity in humans may be produced by constructing a humanised antibody comprising the constant regions of human antibody with the CDR's of a murine human anti-Fas antibody [*c.f.* Jones, P. T., *et al.* (1986), Nature, *321*, 522-525].

The present invention also provides methods and therapeutic compositions for treating the conditions referred to above. Such compositions typically comprise a therapeutically effective amount of the protein of the present invention in admixture with a pharmaceutically acceptable carrier therefor. The composition may be administered in any suitable manner, such as by parenteral, intravenous, subcutaneous or topical administration. In particular, where the condition to be treated is local, then it is preferred to administer the protein as close as possible to the site. For example, serious rheumatic pain may be experienced in major joints, and the protein may be administered at such locations. Systemically administered proteins of the present invention are particularly preferably administered in the form of a pyrogen-free, therapeutically, particularly parenterally, acceptable aqueous solution. The preparation of such pharmaceutically acceptable protein solutions with regard to aspects such as pH, isotonicity, stability and the like, is well within the skill of the person skilled in the art. In addition, the compositions of the present invention may comprise such further ingredients as may be deemed appropriate, such as cell growth retardants and other medicaments.

The dosage regimen for the various conditions treatable with the proteins of the present invention will be readily apparent to one skilled in the art, taking into account various factors, such as the condition, body weight, sex and diet of the patient, the severity of any symptoms, time, the desirability of repeat treatment, as well as any other appropriate clinical factors. As a general guide, the daily dose should typically be in the range of 1 - 1000 μg protein per kilogram of body weight.

The invention will now be explained in more detail with reference to the following Examples, the Examples being illustrative of, but not binding on, the present invention. Any methods, preparations solutions and such like which are not specifically defined may be found in 'Molecular cloning - A Laboratory Handbook' *(supra).* All solutions are aqueous and made up in sterile, deionised water, unless otherwise specified.

**EXAMPLE 1**

**Cloning of DNA Encoding the Variable Region of Mouse Monoclonal Antibody CH11 against the Human Fas Antigen**

**(1-1) Preparation of poly(A)$^+$RNA**

Total RNA was prepared from a CH11-producing hybridoma (obtained from Yonehara, *supra)* in accordance with the method described by Chirgwin and co-workers [see Chirgwin, J. M., *et al.,* (1979) Biochemistry 18:5294]. Specifically, the CH11-producing hybridoma [see Yonehara, S., *et al.,* (1994), International Immunology *6*, 1849-1856] was cultured in ASF104 medium (Ajinomoto) containing 10% (v/v) foetal bovine serum (Gibco). Approximately 6.7 x $10^8$ cells were harvested by centrifugation and the supernatant was discarded. The resulting pellet of cells was then mixed straightaway with 60 ml of 4 M guanidine thiocyanate solution (Fluka). The cells in the resulting suspension were subsequently lysed by aspirating the cell suspension through a syringe equipped with a 21-gauge needle three times. The cell lysate thus obtained was layered onto 3 ml of 5.7 M caesium chloride/0.1 M EDTA solution (pH 7.5) in an ultracentrifugation tube (13PA:Hitachi Koki) and the tube was spun in an Hitachi RPS-40T Rotor (13PA tube, 150,000 x g at 20°C for 18 hours) to precipitate the RNA. The precipitated RNA was dissolved in water, extracted with chloroform/1-butanol (4 : 1, v/v) and then re-precipitated with 100% ethanol.

Poly (A)$^+$ RNA was purified next from the total, resulting RNA, prepared above, by routine methods [*c.f.* Maniatis, T. *et al.,* (1988), "Molecular Cloning: A Laboratory Manual" (2nd Edition), Cold Spring Harbor Lab., 7.26 - 7.28]. More specifically, a disposable polystyrene column (diameter 0.7 cm) was packed with 100 mg of oligo dT cellulose (Pharmacia, Type 7). The column was equilibrated with a loading buffer, comprising 20 mM tris-hydrochloric acid (pH 7.6), 0.5 M sodium chloride, 1 mM ethylenediamine tetraacetate (EDTA) and 0.1% (w/v) sodium dodecylsulphate (SDS). Total RNA (approximately 1.2 mg), was then dissolved in a total volume of 400 μl of water by heating at 65°C for 5 minutes, and then 400 μl of loading buffer (made up at double the above concentration) was added to the solution. The resulting mixture was cooled to room temperature and then poured onto the column. The fraction that passed straight through the column was recovered, heated at 65°C for a further 5 minutes, and poured back onto the column.

The column was next washed with 10 ml of loading buffer, and then further washed with 5 ml of loading buffer containing 0.1 M sodium chloride to remove both non-adsorbates and also non-specific adsorbates. Subsequently, 5 ml of elution buffer [10 mM tris-hydrochloric acid (pH 7.5), 1 mM EDTA and 0.05% (w/v) SDS] was poured onto the column in order to elute specific adsorbates. The resulting eluate was recovered in fractions of 200 μl. The third and fourth 200 μl elution fractions (400 μl in total) were combined, and mixed with 40 μl of 3 M sodium acetate (pH 4.0) and 1 ml of ethanol. The resulting mixture was stored at -20°C overnight. The next day the mixture was spun in a centrifuge (10,000 x g, 4°C for 10 minutes) to recover the pellet. This pellet was used as the poly $(A)^+$ RNA sample and was stored at -80°C until it was required for use.

**(1-2) Cloning of DNA coding for variable regions**

cDNA fragments coding for the variable regions of the H chain and L chain of mouse anti-Fas antigen (CH11) were cloned by 'RT-PCR', which combines reverse transcription (using reverse transcriptase -RT) with the polymerase chain reaction (PCR). The combination of these techniques allowed the specific amplification of a desired sequence from the poly$(A)^+$ RNA sample derived from the CH11-producing hybridoma prepared in (1-1).

Two sets of primers for the RT-PCR reaction were selected from the Ig-Prime Set (Novagen). MuIgV$_H$5'-B and MuIgMV$_H$3'-1 were used to amplify a region of the H chain, while MuIgkV$_L$5' and MuIgMV$_L$3'-1 were used to amplify a region of the L chain. RT-PCR reactions were carried out using both the H chain primer sets and L chain primer sets, respectively.

a) Reverse transcriptase reaction

A reverse transcriptase reaction solution (44 μl) was made up as follows. 10 mM tris-hydrochloric acid (pH 8.3), 50 mM potassium chloride, 0.1 mM dATP, 0.1 mM dGTP, 0.1 mM dCTP, 0.1 mM dTTP, 1.5 mM magnesium chloride, 2.5 pmol of H chain or L chain 3'-side primer, 50 ng of the poly $(A)^+$ RNA prepared in (1.1) and 20 units of reverse transcriptase (BIOCHEMICAL KOGYO CO., LTD.) derived from Moloney murine leukaemia virus (MMLV) were combined and the resulting mixture was incubated at 42°C for one hour.

b) Amplification by PCR

The reverse transcriptase reaction solution prepared in a) was mixed with 25 pmol of H chain or L chain 5' primer, as appropriate, with 5 units of Taq DNA polymerase (ampliTaq DNA Polymerase obtained from Perkin Elmer, Japan) to a final volume of 100 μl of reaction buffer supplied with kit (buffers and solutions for enzymes are as supplied with supplier's kit, unless otherwise specified). The total, resulting, reaction mixture was heated at 94°C for 2 minutes, and then subjected to a heat cycle of 94°C for one minute, 50°C for one minute and 72°C for 2 minutes. This cycle was repeated 30 times. The solution was then kept at 72°C for a further 10 minutes. A gene amplifier PCR system 9600 (Perkin Elmer, Japan) was used to control the reaction temperature in all of the PCR reactions.

c) Assay of PCR product

A portion of the PCR reaction mixture prepared in b) was analysed by gel electrophoresis on a 1.5% (w/v) agarose gel (FMC Bioproducts). The product of each of the H and L chain PCR reactions was obtained as a band of about 430 bp. The band size was estimated by comparing it with molecular weight markers that had also been run on the same gel.

d) Cloning of PCR product

Each of the PCR products obtained in b) was ligated into separate plasmid vectors using an original TA cloning kit (Invitrogen). More specifically, 50 ng of the pCRII vector and four units of T4 DNA ligase (both included in the kit) were added to a ligase reaction buffer [6 mM tris-hydrochloric acid (pH 7.5), 6 mM magnesium chloride, 5 mM sodium chloride, 7 mM β-mercaptoethanol, 0.1 mM ATP, 2 mM dithiothreitol (DTT), 1 mM spermidine and 0.1 mg/ml bovine serum albumin]. The ligase reaction buffer also contained a portion of the PCR reaction mixture which was selected such that it contained approximately 10 ng of the desired PCR product, as estimated by gel electrophoresis in c) above. The resulting mixture was incubated at 14°C for 15 hours.

Subsequently, 2 μl of the ligase reaction mixture was mixed with 50 μl of *E. coli,* strain TOP10F' (included in the kit), which had previously been made competent by the addition of 2 μl of 0.5 M β-mercaptoethanol. The resulting transformation mixture was placed on ice for 30 minutes, heated at 42°C for 30 seconds and then placed on ice again for a further 2 minutes. After this time, the mixture was then added to 500 μl of SOC medium [2% (w/v) tryptone, 0.5% (w/v) yeast extract, 0.05% (w/v) sodium chloride, 2.5 mM potassium chloride, 1 mM magnesium chloride, 20 mM glucose], and the resulting mixture was cultured with rotational shaking for one hour (37°C, 110 rpm). The resulting culture was then spread onto L-broth agar medium plates [1% (w/v) tryptone, 0.5% (w/v) yeast extract, 0.5% (w/v) sodium chloride, 0.1% (w/v) glucose, 0.6% (w/v) Bacto Agar (Difco)] containing 100 µg/ml of ampicillin and the plates were cultured at 37°C overnight without shaking.

Ampicillin-resistant colonies generated by this procedure were selected and scraped off with platinum picks. Cells from the selected colonies were separately cultured in 5 ml of L-broth medium containing 100 µg/ml of ampicillin, at 37°C, overnight. The cultures were then centrifuged to pellet the cells and plasmid DNA was prepared from the cells using the alkaline lysis method (*c.f.* Maniatis, T., *et al., supra).* A plasmid for each of the H and L primer sets was obtained, and these were designated pVH4 (the plasmid containing the fragment amplified using the H chain primer set) and pVL8 (the plasmid containing the fragment amplified using the L chain primer set).

## EXAMPLE 2

## Determination of the Amino Acid Sequence and Nucleotide Sequence of the Variable Regions of CH11

### (2-1) Determination of the N-terminal amino acid sequences of the variable regions of the H chain and L chain of CH11

a) Preparation of CH11

The CH11-producing hybridoma (see Example 1) was grown to a cell number of 2 x $10^8$ in an ASF 104 medium (Ajinomoto) containing 10% (v/v) of bovine serum (Gibco), and this preparation was then cultured in 50 ml of serum free ASF 104 medium at 37°C for 5 days. After this time, the culture was centrifuged (Tommy Seiko's No. 4 rotor, 15,000 x g, 4°C for 15 minutes) and the supernatant was collected. CH11 was obtained from the culture supernatant using an E-Z-Sep antibody purification kit (Pharmacia Biotech).

b) A portion of the purified CH-11, corresponding to 100 µl of the supernatant prepared in a), was added to 10 µl of 100 mM tris-hydrochloric acid buffer (pH 6.8) containing 10% (v/v) of β-mercaptoethanol and 4% (w/v) SDS. The resulting mixture was denatured by heating at 95°C for 5 minutes. The denatured sample was then subjected to electrophoresis on a 12% (w/v) polyacrylamide gel. After electrophoresis the gel was immersed in transfer buffer [25 mM tris-boric acid (pH 9.5), 10% methanol (v/v)] and shaken at room temperature for 15 minutes. The protein bands on the gel were then transferred onto a polyvinylidene difluoride (PVDF) membrane (Nippon Millipore Ltd.), using a semidrive blotting apparatus (Iwaki Glass Co., Ltd.), at a constant current of 0.2 A at 4°C for 1 hour. After this time, the PVDF membrane was stained with a 0.1% (w/v) Coomassie Brilliant Blue solution and destained with 100% methanol. Only two major protein spots were seen, corresponding to the H chain and L chain. These protein spots were excised, and the gel containing them was dried at room temperature.

c) The amino acid sequence of the proteins transferred onto the PVDF membrane in b) was analysed using a gas phase protein sequencer (PPSQ-10; Shimadzu Corporation) using the automatic Edman method [see Edman, P., *et al.,* (1967), Eur. J. Biochem. *1*, 80 *et seq.*]. The N-terminal amino acid sequence of the variable region of the H chain of CH11, and the N-terminal amino acid sequence of the L chain were thus determined, and are shown as SEQ ID NOs. 13 and 14 of the sequence listing, respectively.

### (2-2) Determination of DNA nucleotide sequence

The total nucleotide sequences of the cDNA coding for the variable regions of the H and L chains of CH11 were determined by sequencing the inserts in plasmids pVH4 and pVL8 respectively (prepared in Example 1).

The pCRII vector has an SP6 promoter sequence and a T7 promoter sequence, and these flank any inserted cDNA, thus allowing the sequence of the inserts of pVH4 and pVL8 to be determined using oligonucleotide primers (Perkin Elmer, Japan) corresponding to the sequences. Samples for sequence analysis were prepared using these primers and a dye primer cycle-sequencing kit (Perkin Elmer, Japan). Plasmid DNA from plasmids pVH4 or pVL8 was used as a template. The sequence of each cDNA insert was determined using a DNA sequencer (Model 373, Perkin Elmer, Japan). The cDNA nucleotide sequence of the H chain variable region is shown as SEQ ID NO. 15 and the cDNA nucleotide sequence of the L chain variable region is shown as SEQ ID NO. 16.

The N-terminal amino acid sequence of the H chain of CH11, represented by amino acid Nos. 1 to 15 of SEQ ID NO. 13 in the sequence listing, corresponds completely to the amino acid sequence encoded by nucleotide Nos. 32 to 76 of SEQ ID NO. 15. Therefore, it was deduced that plasmid pVH4 contains DNA coding for the variable region of the H chain of CH11.

The N-terminal amino acid sequence of the L chain of CH11, represented by amino acid Nos. 1 to 21 of SEQ ID NO. 14 in the sequence listing, corresponds completely to the amino acid sequence encoded by nucleotide Nos. 29 to 91 of SEQ ID NO. 16. Therefore, it was deduced that plasmid pVL8 contains DNA coding for the variable region of the L chain of the CH11.

## EXAMPLE 3

## Cloning of DNA Encoding the Complete H Chain, L Chain and J Chains of CH11

### (3-1) Preparation of a cDNA library

A cDNA library was prepared by the Okayama-Berg method [Okayama, H. *et al.,* (1987), Methods in Enzymology *154*, 3-28]. More specifically, 5 μg of poly(A)$^+$ RNA, as prepared in Example 1-1 (a) from the CH11-producing hybridoma, were added to 30 μl of reaction mixture (50 mM tris-hydrochloric acid [pH 8.3], 6 mM magnesium chloride, 40 mM potassium chloride, 2 mM dATP, 2 mM dGTP, 2 mM dCTP, 2 mM dTTP, 2 μg vector primer [3'-oligo(dT)-tailed, pcDV-1]: Pharmacia) containing 75 units of reverse transcriptase (Seikagaku Kogyo, Co., Ltd.) derived from Avian myeloblastosis virus (AMV). The resulting mixture was incubated at 37°C for 30 minutes.

After this time, an equivalent volume of phenol-chloroform (1 : 1, v/v) was added to the reaction mixture and thoroughly mixed. The resulting mixture was centrifuged (10,000 x g, room temperature, 5 minutes) and the aqueous layer was recovered (this procedure of extracting with phenol:chloroform and recovering the aqueous supernatant is referred hereinafter as "phenol-chloroform extraction"). To the resulting aqueous layer were added 35 μl of 4 M ammonium acetate and 140 μl of ethanol, and the resulting mixture was cooled at -70°C for 15 minutes, then centrifuged (10,000 x g, 4°C, 15 minutes). The pellet was washed with a 75% (v/v) solution of ethanol and then dried under reduced pressure.

The dried precipitate was then dissolved in 13 μl of distilled water, and then 5.6 μl of terminal transferase reaction mixture [140 mM sodium cacodylate, 30 mM tris-hydrochloric acid (pH 6.8), 1 mM cobalt chloride, 0.5 mM DTT, 0.3 μg polyadenylic acid (polyA, Pharmacia), 0.2 mM dCTP] was added and the resulting reaction mixture was incubated at 37°C for 5 minutes. Terminal deoxynucleotidyl transferase (21 units, Pharmacia) was then added, in accordance with the supplier's instructions, and the reaction was allowed to proceed for 5 minutes. The reaction mixture was then subjected to phenol-chloroform extraction. Then, 20 μl of 4 M ammonium acetate and 80 μl of ethanol were added to the recovered aqueous layer, and the mixture was cooled at -70°C for 15 minutes, then centrifuged (10,000 x g at 4°C for 15 minutes). The pellet was washed with a 75% (v/v) solution of ethanol and dried under reduced pressure.

The DNA precipitate obtained in this way was dissolved in 30 μl of reaction mixture [10 mM tris-hydrochloric acid (pH 7.5), 60 mM sodium chloride, 7 mM magnesium chloride], and 30 units of restriction enzyme HindIII were added to the resulting solution. In general, where a restriction enzyme is used, but no buffer is specified, then the buffer which is used is the buffer supplied with the enzyme. In the case where DNA is digested with two enzymes, digestion is carried out with the two enzymes sequentially. After the first digestion, the DNA is precipitated, resuspended and then digested with the second enzyme. Precipitation and resuspension techniques are well known in the art (*c.f.* Maniatis *et al., supra).* All of the restriction enzymes and buffers used in the present Examples were supplied by Takara Schuzo.

The DNA was allowed to be digested at 37°C overnight in the digestion solution. Subsequently, the reaction mixture was subjected to phenol-chloroform extraction. Then, 35 μl of 4 M ammonium acetate and 140 μl of ethanol were added to the recovered aqueous layer, and the mixture was cooled at -70°C for 15 minutes. The mixture was centrifuged (10,000 x g, 4°C x 15 minutes) to precipitate the DNA, and the resulting pellet was washed with a 75% (v/v) solution of ethanol and dried under reduced pressure. The thus prepared precipitate DNA was used as a cDNA sample in subsequent procedures.

In parallel, plasmid DNA from the vector pcDL-SRα296 [*c.f.* Takebe, Y *et al.,* (1989), "JIKKEN IGAKU (Experimental Medicine)", *7*, pp. 95-99] was digested with the restriction enzyme PstI. The product of the digestion was treated with dGTP and terminal deoxynucleotidyl transferase (Pharmacia), in order to add oligo dG to the 3' terminal end, as follows:

pcDL-SRα296 DNA (100μg, present in 50μl) was added to 10μl of 10x terminal deoxynucleotidyl transferase buffer [1x buffer: 1.4 M sodium cacodylate, 0.3M Tris-HCl, (pH 7.6), 10μl of DTT (1mM), 20μl of 0.1mM $^3$H-dGTP (Dupont) and 10μl of terminal transferase (210 IU, Pharmacia)]. The mixture was incubated for 40 minutes at 37°C, and then mixed with an equal volume of TE buffer-saturated phenol. After standing, the aqueous layer was removed and subjected to a phenol-chloroform extraction. After both of the phenol and phenol-chloroform extractions had been performed, then the DNA was precipitated using ethanol and resuspended in 50μl of TE buffer.

The total precipitated DNA was digested with the restriction enzyme HindIII, and the products of the digestion were separated by gel electrophoresis on a 1.8% (w/v) agarose gel. A band of 800 bp was excised from the gel, and extracted from the gel using a GENECLEAN kit (Funakoshi) according to the manufacturer's instructions. The resulting DNA was dissolved in 100 μl of TE buffer, and 100μl of ethanol was added. The final concentration of the DNA was 0.09 μg/μl.

The resulting product yielded a linker-DNA in which oligo (dG) is attached to the SRα promoter (*c.f.* Figure 1, which is a schematic view of the construction of a cDNA library to enable cloning of DNA encoding the total length of each subunit of CH11. Figure 2 is a diagram showing the process of cloning and amplifying DNA encoding the total length of each subunit of CH11).

The precipitated, dried, cDNA sample, prepared above, was dissolved in 10 μl TE buffer [10 mM tris-hydrochloric acid (pH 7.5), 1 mM EDTA]. A portion of the resulting solution (1 μl) was added to reaction buffer [10 mM, tris hydrochloric acid (pH 7.5), 1 mM EDTA, 100 mM sodium chloride] containing 0.08 pmol of the linker-DNA prepared above. The resulting mixture was heated at 65°C for 5 minutes and then incubated at 42°C for 30 minutes. After this time, 10 μl of 10x ligase buffer [10 mM ATP, 660 mM tris-hydrochloric acid (pH 7.5), 66 mM magnesium chloride, 100 mM DTT], 76 μl of distilled water and 1 μl of 10 m M β-nicotinamide adenine dinucleotide (NAD, Boehringer Mannheim) were added to the reaction mixture, and the resulting mixture was cooled on ice for 10 minutes. *E. coli* DNA ligase (8.4 μg equivalent, Pharmacia) was then added to the cooled reaction mixture, and the whole was incubated at 12°C, overnight.

After this incubation, 2 μl of nucleotide solution (2 mM dATP, 2 mM dCTP, 2 mM dGTP, 2 mM dTTP), 0.5 μl of 10 mM NAD, 42 μg equivalent of *E. coli* DNA ligase (Pharmacia), 4.1 units of DNA polymerase I (Pharmacia), and 5.5 units of ribonuclease H (Pharmacia) were added to the reaction mixture. The resulting mixture was then incubated at 12°C for one hour and then at 22°C for a further hour. The cDNA library prepared in this way was stored at -20°C until it was needed.

**(3-2) <u>Cloning by PCR</u>**

a) Preparation of primer

In the case of the H chain, the amino acid sequence of the variable region of the H chain of CH11, as determined in Example 2, was compared with the antibody amino acid sequence database prepared by Kabat *et al.* [see Kabat E. A. *et al.,* (1991), in "Sequences of Proteins of Immunological Interest Vol. II", U. S. Department of Health and Human Services]. It was determined that the H chain (μ chain) of CH11 was sub class 2A. Therefore, an oligonucleotide primer was synthesised such that it would hybridise with a part of the 5'-non-translated region of the DNA coding for mouse H chain, sub class 2a. The oligonucleotide primer which was selected had the sequence: 5'-CTAAGGGAAT TCCGCCTCTC CTCAGACACT GAA-3' (H5-1; SEQ ID NO. 17 of the sequence listing).

An oligonucleotide primer was also designed that would hybridise with a part of the 3' non-translated region of the CH11 H-chain. The design of the olignucleotide was based on the nucleotide sequence of the DNA coding for the mouse immunoglobulin M chain constant region reported by Goldberg, *et al.* [see Goldberg, I.G., *et al.,* (1981), Gene *15,* 33-42], and the sequence which was selected was: 5'-TTTTACTCTA GAGACCCAAG GCCTGCCTGG TTGA-3' (H3-1; SEQ ID NO. 18 of the sequence listing).

For the L chain, the amino acid sequence of the variable region of the L chain of CH11, as determined in Example 2, was compared with the antibody amino acid sequence database prepared by Kabat and co-workers *(supra).* It was found that the L chain of CH11 was sub-class κ2. Therefore, an oligonucleotide primer was designed such that it would hybridise with a part of the 5'-terminal, non-translated region of the DNA coding for mouse L chain, sub-class κ2. The oligonucleotide primer which was selected had the sequence: 5'-AAATAGGAAT TCCAGTCTCC TCAGGCTGTC TCC-3' (L5-1; SEQ ID NO. 19 of the sequence listing).

An oligonucleotide primer was also designed that would hybridise with a part of the 3' non-translated region. The design of the olignucleotide was based on the nucleotide sequence of the DNA coding for the mouse immunoglobulin κ chain constant region registered under the registration name MUSIGB1L1 (Accession No. D14630). The sequence used was: 5'-ATGATCTCTA GAGTGGTGGC ATCTCAGGAC CT-3' (L3-1; SEQ ID NO. 20 of the sequence listing).

In the case of the J chain, there is no variable region and both the sequence of the DNA coding for the J chain, and the amino acid sequence of the J chain are known [*c.f.* Cann, G.M., *et al.,* (1982), Proc. Natl. Acad. Sci. USA, *79,* 6656-6660]. Based on this finding, oligonucleotide primers were synthesised that would hybridise with a part of the 5' and 3' non-translated regions of the DNA coding for the J chain. These oligonucleotides had the sequences: 5'-TTGCGGAATT CCTCACCTGT CCTGGGGTTA TT-3' (J5-1; SEQ ID NO. 21 of the sequence listing) and 5'-ATTGCCTCTA GAGCCTCTAA GGACAACGAC CT-3' (J3-1; SEQ ID NO. 22 of the sequence listing).

These oligonucleotide primers were all synthesised using an automatic DNA synthesiser 380 B (Perkin Elmer, Japan) by the phosphoamidite method [see Mattrucci, M. D. and Caruthers, M. H. (1981), J. Am. Chem. Soc., *103,* 3185-3191]. After synthesis was complete, each primer was cleaved from the support and deprotected, and then freeze dried. The resulting product was dissolved in distilled water and stored at -20°C until it was needed.

b) Amplification of target gene by PCR

PCR reaction solution [10 mM tris-hydrochloric acid (pH 8.3), 50 mM potassium chloride, 1.5 mM magnesium chloride, 2.5 mM dATP, 2.5 mM dGTP, 2.5 mM dCTP, 2.5 mM dTTP] was prepared, and 0.1 µl of the cDNA library described in Example 4-1, 1 unit of Taq DNA polymerase (Perkin Elmer, Japan) and 15 pmol of the oligonucleotide primer (prepared in 3-2 a) were added to 100 µl of the PCR reaction solution and heated at 94°C for 2 minutes. The resulting mixture was then subjected to a heat cycle of 94°C for one minute, 55°C for one minute and 72°C for 2 minutes. This cycle was repeated 30 times. After the last cycle, the solution was kept at 72°C for a further 10 minutes.

The combinations of the primers that were used in the respective reactions are as follows:

H5-1 and H3-1 (for H chain);
L5-1 and L3-1 (for L chain); and
J5-1 and J3-1 (for J chain).

c) Assay of PCR product

After the PCR reaction in b) had been performed, a portion of the reaction mixture was analysed by gel electrophoresis on a 0.8% (w/v) agarose gel in the case of the H chain. For the L and J chains, a 1.5% (w/v) agarose gel (agarose was obtained from FMC Bioproducts) was used. The product of the PCR reaction was a band of approximately 1900 bp for the H chain, 800 bp for the L chain and 650 bp for the J chain. The band sizes were estimated by comparison with molecular weight markers run on the same gel

d) Cloning of PCR product

Each of the PCR products obtained in b) was ligated into a plasmid vector, using a eukaryote TA cloning kit (Invitrogen). More specifically, 60 ng of pCR3 vector (included in the kit) and four units of T4 DNA ligase were added to ligase reaction buffer [6 mM tris-hydrochloric acid (pH 7.5), 6 mM magnesium chloride, 5 mM sodium chloride, 7 mM β-mercaptoethanol, 0.1 mM ATP, 2 mM DTT, 1 mM spermidine, 0.1 mg/ml bovine serum albumin], containing a portion of the PCR reaction mixture. The volume of the PCR reaction mixture was selected such that it contained about 10 ng of the desired PCR product, as estimated by gel electrophoresis. The resulting mixture was incubated at 14°C for 15 hours.

A portion of the ligase reaction mixture (2µl) was mixed with 50 µl of *E. coli* cells, strain TOP10F' (included in the kit), made competent by the addition of 2 µl of 0.5 M β-mercaptoethanol. The resulting mixture was placed on ice for 30 minutes, warmed at 42°C for 30 seconds, then placed on ice again for 2 minutes. SOC medium (500 µl, as described above) was then added to this mixture, and the resulting mixture was cultured at 37°C with rotational shaking (110 rpm) for one hour. The culture liquid was then spread onto L-broth agar medium plates containing 100 µg/ml of ampicillin and cultured at 37°C overnight. Ampicillin-resistant colonies which appeared were then scraped off with a platinum pick and cultured in 5 ml of L-broth medium containing 100 µg/ml of ampicillin at 37°C overnight. These cultures were centrifuged to precipitate cells which were then used to prepare plasmid DNA by the alkaline lysis method (Maniatis *et al., supra*).

Three of the resulting plasmids were designated pCR3-H123 (the plasmid including H chain-coding cDNA), pCR3-L103 (the plasmid including L chain-coding cDNA) and pCR3-J1123 (the plasmid including J chain-coding cDNA). Competent cells of *E. coli* strain DH5α (Gibco) were transformed with one of the plasmids pCR3-H123, pCR3-L103 or pCR3-J1123 and the resulting transformants were deposited at the Research Institute of Life Science and Technology of the Agency of Industrial Science and Technology on February 28, 1996 under the deposit Nos. FERM BP-5427, FERM BP-5428 and FERM BP-5429, respectively. DNA encoding the H, L and J chains of CH11 are readily prepared from these strains by well known methods.

## EXAMPLE 4

## Determination of Total Nucleotide Sequence of the cDNA Coding for CH11 H Chain, L Chain and J chains

### (4-1) Determination of nucleotide sequence of DNA

The mouse immunoglobulin M chain consists of an N-terminal variable region containing about 110 residues and a constant region containing about 470 residues, adjacent to the variable region. The mouse immunoglobulin K chain consists of an N-terminal variable region containing about 110 residues and a constant region containing 107 residues adjacent the variable region. It was predicted that the complete nucleotide sequences of the cDNAs coding for the CH11 H chain and L chain would consist of nucleotide sequences coding for known constant regions and which were ligated to nucleotide sequences coding for the variable regions of the chains, as identified in Example 2 [*c.f.* Kabat E. A. *et. al.,* (1991), in "Sequences of Proteins of Immunological Interest Vol. II", U.S. Department of Health and Human Services].

The nucleotide sequence encoding the J chain of CH11 was presumed to be the same as that of the known J chain sequence.

Based on these presumed nucleotide sequences, oligonucleotide primers of 20 nucleotides in length were synthesised, corresponding to sequences of the H, L and J chains, separated by coding intervals of 60 to 200 bp. These primers were used for sequence analysis.

The sequences of the synthesised oligonucleotide primers were as follows:

For the H chain:

5'-TGGGGCCTCA GTGAAGATAT -3' (SHF-2; SEQ ID NO. 23 of the sequence listing)

5'-CAATGGTGGT ACTGGCTACA -3' (SHF-3; SEQ ID NO. 24 of the sequence listing)

5'-TGACATCTGA GGACTCTGCA -3' (SHF-4; SEQ ID NO. 25 of the sequence listing)

5'-TCCTCAGAGA GTCAGTCCTT -3' (SHF-6; SEQ ID NO. 26 of the sequence listing)

5'-TCCTTCACCT GGAACTACCA -3' (SHF-7; SEQ ID NO. 27 of the sequence listing)

5'-TCCCAAGAGC ATCCTTGAAG -3' (SHF-8; SEQ ID NO. 28 of the sequence listing)

5'-AGATCTGCAT GTGCCCATTC -3' (SHF-9; SEQ ID NO. 29 of the sequence listing)

5'-TCTAAACTCA TCTGCGAGGC -3' (SHF-10; SEQ ID NO. 30 of the sequence listing)

5'-GGTGACCATC GAGAACAAAG -3' (SHF-11; SEQ ID NO. 31 of the sequence listing)

5'-AGGGGTCTCA CCTTCTTGAA -3' (SHF-12; SEQ ID NO. 32 of the sequence listing)

5'-TCCTTTGCCG ACATCTTCCT -3' (SHF-13; SEQ ID NO. 33 of the sequence listing)

5'-GTGTGTACTG TGACTCACAG -3' (SHF-15; SEQ ID NO. 34 of the sequence listing)

5'-AACTGAACCT GAGGGAGTCA -3' (SHF-16; SEQ ID NO. 35 of the sequence listing)

5'-AACTCTTGCC CCAAGAGAAG -3' (SHF-17; SEQ ID NO. 36 of the sequence listing)

5'-ATCCTGACTG TGACAGAGGA -3' (SHF-18; SEQ ID NO. 37 of the sequence listing)

5'-ACAAGTCCAC TGGTAAACCC -3' (SHF-19; SEQ ID NO. 38 of the sequence listing)

5'-AGGATATCTT CACTGAGGCC -3' (SHR-1; SEQ ID NO. 39 of the sequence listing)

5'-ATCCACTCAA GGCTCTTTCC -3' (SHR-2; SEQ ID NO. 40 of the sequence listing)

5'-ACTGCAGAGT CCTCAGATGT -3' (SHR-3; SEQ ID NO. 41 of the sequence listing)

5'-AGACGGTGAC TGAGGTTCTT -3' (SHR-4; SEQ ID NO. 42 of the sequence listing)

5'-CAGGTGAAGG AAATGGTGCT -3' (SHR-5; SEQ ID NO. 43 of the sequence listing)

5'-ATGCTCTTGG GAGACAGCAA -3' (SHR-6; SEQ ID NO. 44 of the sequence listing)

5'-CTCTGTTTTT GCCTCCGTAG -3' (SHR-7; SEQ ID NO. 45 of the sequence listing)

5'-TGGCCTCGCA GATGAGTTTA -3' (SHR-8; SEQ ID NO. 46 of the sequence listing)

5'-CCTTTGTTCT CGATGGTCAC -3' (SHR-9; SEQ ID NO. 47 of the sequence listing)

5'-TGTGGAGGAC ACGTTCTTCA -3' (SHR-10; SEQ ID NO. 48 of the sequence listing)

5'-ACTTTGAGAA GCCCAGGAGA -3' (SHR-12; SEQ ID NO. 49 of the sequence listing)

5'-AGATCCCTGT GAGTCACAGT -3' (SHR-13; SEQ ID NO. 50 of the sequence listing)

5'-AGCAGGTGGA TGTTTGTGCA -3' (SHR-14; SEQ ID NO. 51 of the sequence listing)

5'-TGAAGCCACT GCACACTGAT -3' (SHR-15; SEQ ID NO. 52 of the sequence listing)

5'-AGTTCCATTC CTCCTCTGTC -3' (SHR-16; SEQ ID NO. 53 of the sequence listing)

5'-TGTGTCAGAC ATGATCAGGG -3' (SHR-18; SEQ ID NO. 54 of the sequence listing)

For the L chain:

5'-TGAAGTTGCC TGTTAGGCTG -3' (SLF-1; SEQ ID NO. 55 of the sequence listing)

5'-CTTGGAGATC AAGCCTCCAT -3' (SLF-2; SEQ ID NO. 56 of the sequence listing)

5'-GCTGAGGATC TGGGAGTTTA -3' (SLF-3; SEQ ID NO. 57 of the sequence listing)

5'-GATGCTGCAC CAACTGTATC -3' (SLF-4; SEQ ID NO. 58 of the sequence listing)

5'-CGACAAAATG GCGTCCTGAA -3' (SLF-5; SEQ ID NO. 59 of the sequence listing)

5'-ACGTTGACCA AGGACGAGTA -3' (SLF-6; SEQ ID NO. 60 of the sequence listing)

5'-ATCTGCAAGA GATGGAGGCT -3' (SLR-2; SEQ ID NO. 61 of the sequence listing)

5'-ACCCCAGAAA ATCGGTTGGA -3' (SLR-3; SEQ ID NO. 62 of the sequence listing)

5'-CCGGAGGAAC ATGTGTACTT -3' (SLR-4; SEQ ID NO. 63 of the sequence listing)

5'-TCGTTCATAC TCGTCCTTGG -3' (SLR-6; SEQ ID NO. 64 of the sequence listing)

5'-CATCTCAGGA CCTTTGTCTC -3' (SLR-7; SEQ ID NO. 65 of the sequence listing)

For the J chain:

5'-CACCTGTCCT GGGGTTATTT -3' (SJF-1; SEQ ID NO. 66 of the sequence listing)

5'-AGACAAGATG AAGACCCACC -3' (SJF-2; SEQ ID NO. 67 of the sequence listing)

5'-AAGCGACCAT TCTTGCTGAC -3' (SJF-3; SEQ ID NO. 68 of the sequence listing)

5'-ATATCTCTGA TCCCACCTCC -3' (SJF-8; SEQ ID NO. 69 of the sequence listing)

5'-GAAATGCGAT CCTGTGGAAG -3' (SJF-5; SEQ ID NO. 70 of the sequence listing)

5'-CTATACCACT ATGGTCCCAC -3' (SJF-6; SEQ ID NO. 71 of the sequence listing)

5'-AGAAGCAGGT GGGTCTTCAT -3' (SJR-2; SEQ ID NO. 72 of the sequence listing)

5'-TAGAGGTAAC TCGGGTACAC -3' (SJR-3; SEQ ID NO. 73 of the sequence listing)

5'-AAGTTCCTTC TCAGTGGGGA -3' (SJR-8; SEQ ID NO. 74 of the sequence listing)

5'-GGTGGCAGTA ACAACCTGAT -3' (SJR-5; SEQ ID NO. 75 of the sequence listing)

5'-CATGATACCT AAGTGGGACC -3' (SJR-6; SEQ ID NO. 76 of the sequence listing)

Each oligonucleotide primer was synthesised by the phosphoamidite method using an automatic DNA synthesiser (Model 380B: Perkin Elmer, Japan). Samples for sequence analysis of the H chain were prepared using DNA from plasmid pCR3-H123. Samples for sequence analysis of the L chain were prepared using DNA from plasmid pCR3-L103. Samples for sequence analysis of the J chain were prepared using DNA from pCR3-J1123. The PCR reaction was carried out using a Prism Ready Reaction Terminator Cycle Sequencing Kit (Perkin Elmer, Japan), as follows.

pCR3-H123 (1.5 μg) and 4.8 pmol of primer (SHF-2) were mixed to a final volume of 16 μl in distilled water. A portion of this pCR3-H123/primer mixture (9.5 μl) was mixed with 10.5 μl of a premix containing Taq DNA polymerase. All of this procedure was in accordance with the instructions in the kit. The resulting mixture was placed in an automatic reactor (Catalyst: Perkin Elmer, Japan). The reaction cycle used was as follows: 95°C for 30 seconds, 50°C for 15 seconds and 60°C for 4 minutes, repeated 25 times.

After completion of the reaction cycles, 80 μl of sterilised water was added to the resulting solution, and the DNA in the resulting mixture was extracted twice by the phenol/chloroform method. The recovered aqueous layer was mixed with 15 μl of 2 M sodium acetate and 300 μl of ethanol, followed by centrifugation to recover the precipitate. The precipitate was washed with a 70% (v/v) solution of ethanol and dried under reduced pressure, then dissolved in 3 μl of sample solution (4 μl 0.25 M EDTA, 100 μl formamide and 15 μl sterilised water).

Sequencing reactions were run and analysed on a DNA sequencer (Model 373A: Perkin Elmer, Japan), for the 32 H chain primers, the 11 L chain primers and the 11 J chain primers.

The sequence data obtained for each primer were combined and integrated in order to determine the complete nucleotide sequence of the H, L and J chains of CH11. The cDNA nucleotide sequences of each plasmid insert are shown by SEQ ID NOs. 7, 9 and 11 of the sequence listing, respectively. The amino acid sequences that correspond to these nucleotide sequences are shown by SEQ ID NOs. 8, 10 and 12 of the sequence listing, respectively.

**(4-2) Primary structure of the H chain of CH11**

The nucleotide sequence of the H chain variable region, shown as nucleotide Nos. 32 to 379 of SEQ ID NO. 15 of the sequence listing, was found to be identical with that of the nucleotide Nos. 58 to 405 of SEQ ID NO 7.

The amino acid sequence shown as amino acid Nos. 117 to 571 of SEQ ID NO. 8 was found to be identical with the amino acid sequence in the H chain constant region derived from mouse IgM, when compared with the database of antibody amino acid sequences [Kabat E.A. *et. al., supra*).

The amino acid sequence shown as amino acid Nos. -19 to -1 of SEQ ID NO. 8 was concluded to be a signal sequence of the H chain of CH11.

The nucleotide sequence shown as nucleotide Nos. 406 to 1770 of SEQ ID NO. 7 was found to be identical with that of the H chain constant region of mouse IgM.

Based on these results, the total nucleotide sequence could be established, together with the total amino acid sequence.

**(4-3) Primary structure of CH11 L chain**

The nucleotide sequence of the L chain variable region, shown as nucleotide Nos. 29 to 364 of SEQ ID NO. 16 in the sequence listing, was found to be identical with that of nucleotide Nos. 58 to 393 of SEQ ID NO. 9.

The amino acid sequence shown as amino acid Nos. 113 to 219 of SEQ ID NO. 10 was found to be identical with the amino acid sequence in the mouse κL chain constant region, when compared with Kabat's database of antibody amino acid sequences.

The amino acid sequence shown as amino acid Nos. -19 to -1 of SEQ ID NO. 10 was concluded to be a signal sequence for the L chain.

The nucleotide sequence shown as nucleotide Nos. 394 to 714 of SEQ ID NO. 9 was established to be completely identical with that in the mouse κL chain constant region.

Based on these results, the total nucleotide sequence could be established, together with the total amino acid sequence.

**(4-4) Primary structure of J chain of CH11**

The amino acid sequence shown as amino acid Nos. 1 to 137 of SEQ ID NO. 12 was compared with the antibody amino acid sequence database, and found to be identical to the known mouse J chain.

The nucleotide sequence shown as nucleotide Nos. 67 to 477 of SEQ ID NO. 11 was found to be identical with that of the known mouse J chain.

The amino acid sequence shown as amino acid Nos. -22 to -1 of SEQ ID NO. 12 was concluded to be a signal sequence for the J chain of CH11.

Based on these results, the total nucleotide sequence could be established, together with the total amino acid sequence.

**(4-5) Determination of Complementarity Determining Regions (CDR)**

Both the position and the amino acid sequence of each CDR in the variable regions of the H chain and L chain of CH11, determined as described above, were identified by comparison with Kabat's antibody amino acid sequence database *(supra).* This database shows that the amino acid chain length of the framework area in the variable region is substantially constant throughout different antibodies, provided that the sub-type is the same, and provided that that the amino acid sequences have some common characteristics. However, the CDR's, present between such framework regions, are sequences specific to each antibody.

By comparison of the amino acid sequence of the variable region of CH11 H chain with the sequence of mouse μ2a sub type, the CDR in the CH11 H chain was shown to be represented by amino acid Nos. 31 to 35 of SEQ ID NO. 8 ($CDRH_1$, corresponding to SEQ ID NO. 1 of the sequence listing), 50 to 66 of SEQ ID NO. 8 ($CDRH_2$, corresponding to SEQ ID NO. 2 of the sequence listing) and 99 to 105 of SEQ ID NO. 8 ($CDRH_3$, corresponding to SEQ ID NO. 3 of the sequence listing).

When the amino acid sequence of the variable region of CH11 L chain was compared to the sequence of the mouse κ2 sub-type, the CDR of the L chain was shown to be represented by the amino acid Nos. 24 to 39 of SEQ ID NO. 10 ($CDRL_1$, corresponding to SEQ ID NO. 4 of the sequence listing), 55 to 61 of SEQ ID NO. 10 ($CDRL_2$, corresponding to SEQ ID NO. 5 of the sequence listing) and 94 to 102 of SEQ ID NO. 10 ($CDRL_3$, corresponding to SEQ ID NO. 6 of the sequence listing).

When the amino acid sequence of the variable region of CH11 L chain was compared to the sequence of the mouse κ2 sub-type, the CDR of the L chain was shown to be represented by the amino acid Nos. 24 to 39 ($CDRL_1$, corresponding to SEQ ID NO. 4 of the sequence listing), 55 to 61 ($CDRL_2$, corresponding to SEQ ID NO. 5 of the sequence listing) and 94 to 102 ($CDRL_3$, corresponding to SEQ ID NO. 6 of the sequence listing) of SEQ ID NO. 10 of the sequence listing.

## EXAMPLE 5

## Expression of the Genes Coding for each Sub Unit of CH11 in COS-1 Cells

### (5-1) Construction of expression vector

DNA from the high-expression vector pME18S [see Hara, T., *et al.,* (1992), EMBO J., *11*, 1875 *et seq.*] was digested with the restriction enzymes EcoRI and Xba1. The resulting digestion products were separated by agarose gel electrophoresis on a 0.8% agarose gel. Following electrophoresis the gel was stained with ethidium bromide (0.25 μg/ml) to stain the DNA. A band of approximately 3000 bp was excised from the gel and transferred to a dialysis tube (permeation limit: 12000 to 14000 Da, Gibco) along with 250 μl of TBE agarose gel electrophoresis buffer (TBE buffer: Tris 0.089M, Borate 0.089M, EDTA (pH 8.0) 0.002M) and the tube was sealed. The sealed tube was placed in a submarine type electrophoresis tank containing more of the agarose gel electrophoresis buffer and the DNA was electrophoretically eluted from the gel (150 V, 15 minutes). The dialysis tube was taken out, and the DNA eluate in the tube was recovered and subjected to phenol/chloroform extraction.

In parallel, plasmid pCR3-H123, prepared as described in Example 3 d), and which contained the DNA coding for the CH11 H chain, was digested with the restriction enzymes EcoR1 and Xba1. The 1900 bp EcoRI-XbaI fragment was isolated and purified by agarose gel electrophoresis on a 0.8% gel, as described in 5-1 above. This cDNA fragment was ligated to the 3 kb EcoRI-XbaI fragment of pME18S, isolated as above, using DNA ligation kit version 1 (Takara Shuzo Co., Ltd.).

Plasmid pCR3-L103, prepared in Example 3, containing the DNA coding for the CH11 L chain, was digested with the restriction enzymes EcoR1 and Xba1. An 840 bp EcoRI-XbaI fragment containing the cDNA plasmid insert was isolated and purified by agarose gel electrophoresis on a 0.8% gel, as described in 5-1 above. This cDNA fragment was ligated to the 3 kb EcoRI-XbaI fragment of pME18S obtained above, using DNA ligation kit version 1 (Takara Shuzo Co., Ltd.).

Plasmid pCR3-J1123 prepared in Example 3, containing the DNA coding for the CH11 J chain, was digested with the restriction enzymes EcoR1 and Xba1. A 640 bp EcoRI-XbaI fragment containing the cDNA plasmid insert was isolated and purified by agarose gel electrophoresis on a 0.8% gel, as described in 5-1 above. This cDNA fragment was ligated to the 3 kb EcoRI-XbaI fragment of pME18S obtained above, using DNA ligation kit version 1 (Takara Shuzo Co., Ltd.).

After the ligation reactions had been completed, JM109 *E. coli* competent cells (Takara Shuzo) were transformed using each reaction mixture, and colonies were selected for ampicillin-resistant strains. Plasmid DNA was prepared from the transformed strains by alkaline lysis (Maniatis, *supra*). The resulting plasmid DNA was digested with EcoR1 and Xba1, which was then followed by agarose gel electrophoresis on a 0.8% gel, to confirm that DNA of the desired size had been cloned.

The expression vector containing the DNA coding for the CH11 H chain was designated pHEX126. The expression vector containing the DNA coding for the CH11 L chain was designated pLEX004, and expression vector containing the DNA coding for the CH11 J chain was designated pJEX004.

### 5-2 Expression in COS-1 cells

COS-1 cells were transformed with pHEX126, pLEX004 and pJEX004 by electroporation using the GTE-1 apparatus (Shimadzu Corporation). More specifically, COS-1 cells (American Type Culture Collection No. CRL-1650) were cultured in a $225cm^2$ culturing flask (Sumitomo Bakelite). The cells were grown to a semiconfluent state in Dulbecco's modified Eagle's minimum essential medium ('DMEM', Nissui Pharmaceutical) containing 10% (v/v) foetal bovine serum (CSL). The medium was removed and the cells were treated twice with 3 ml of trypsin-EDTA solution (Sigma) at 37°C for 3 minutes. The cells were recovered, then washed twice with a phosphate buffered saline (PBS, pH 8.0) buffer (Nissui Pharmaceutical). The washed COS-1 cells were adjusted to a density of $6 \times 10^7$ cells/ml with PBS (-) buffer (8g NaCl, 0.2g KCl, 1.15g $Na_2HPO_4$, 0.2g $KH_2PO_4$ per litre, Nissui Seiyaku Co.).

DNA was prepared from each of the pHEX126, pLEX004 and pJEX004, which encode all three of the sub units of CH11, using a plasmid maxiprep kit (Maxiprep DNA purification system; Promega). A portion (20 µg) of each plasmid maxiprep was precipitated using ethanol, and the precipitate was dissolved in 20 µl of PBS (-) buffer. The DNA prepared in this way was used for transfection of the COS-1 cells. Any transfections carried out using more than one plasmid used 20 µg of each plasmid.

A suspension of COS-1 cells (20 µl; 1.2 x $10^6$ cells) prepared in this way was mixed with 20 µl of each sample, and the resulting mixture was placed in an electroporation cuvette (Shimadzu Corporation) having an electrode interval of 2 mm. The cuvette was placed into the electroporation apparatus and two 600V-30 µsec pulses were applied, with a one second interval between pulses. The mixture of the cells and DNA was added to 10 ml of DMEM containing 10% (v/v) foetal bovine serum, then transferred to a cell culturing plastic dish (diameter: 90 mM; Sumitomo Bakelite) and cultured at 37°C under 7.5% $CO_2$ for 24 hours. After this time the culture supernatant was removed and the cells were washed with a serum-free DMEM medium. Fresh serum-free DMEM medium was added (10 mls), and culturing continued at 37°C under 7.5% $CO_2$ for 24 hours. The culture supernatant was then recovered.

The COS-1 cells were transfected using either one plasmid, or a combination of plasmids, as listed below. The culture supernatant was recovered in each case.

| | |
|---|---|
| [A]: | pME18S only |
| [B]: | pHEX126 only |
| [C]: | pLEX004 only |
| [D]: | pJEX004 only |

| | |
|---|---|
| [E]: | pHEX126 and pLEX004 |
| [F]: | pHEX126, pLEX004 and pJEX004 |

## EXAMPLE 6

### Detection of anti-Fas Antibody

The anti-Fas antibody prepared according to the present invention was detected by two methods, separately.

(1) The first method was Western Blotting. The protein sample was run on an SDS-polyacrylamide gel under the conditions shown below, and the sample proteins separated by electrophoresis (this whole process is known as SDS polyacrylamide gel electrophoresis or 'SDS-PAGE'). The proteins were transferred to a nitrocellulose membrane, and reacted with an antibody specific to the anti-Fas antibody. Cross reaction of the antibody with the target anti-Fas protein is detectable, in the presence of suitable substrates, by light emission on a photographic film.

(2) The second method measures binding affinity to the Fas antigen.

### (6-1) Western blotting

The test sample of culture supernatant (1 ml) was dialysed against 5 litres of pure water, then dried under reduced pressure using a centrifugal concentrator (CC-101; Tomy Seiko). The resulting product was added to 10 µl of a sample buffer [2% (w/v) SDS (electrophoresis grade; Bio-Rad), 5% (v/v) β-mercaptoethanol (Sigma), 10% (v/v) glycerol, 0.1% (w/v) bromophenol blue] and mixed thoroughly. The resulting suspension was heated at 100°C for 5 minutes to provide a sample suitable for electrophoresis. The entire electrophoresis sample was loaded into one well of a SDS-polyacrylamide gel (4 to 20% gradient gel, buffer composition per litre: Tris 3.0g, Glycine 14.4g, SDS 10g, pH 8.3), and the gel was run at a constant current of 20 mA (room temperature, one hour).

Once the electrophoresis had been performed, proteins were transferred from the gel to a nitrocellulose membrane, using the semi-dry blotting method [see Towbin, H, *et al.,* (1979), Proc. Natl. Acad. Sci. USA, *76*, 4350 *et seq.*]. The specific apparatus and conditions used were as follows:

| | |
|---|---|
| Transfer buffer: | 20 mM tris, 150 mM glycine, 10% (v/v) methanol |
| Blotting equipment: | manufactured by Iwaki Glass |
| Operating conditions: | 4°C, 0.2 A (constant current), 1 hour. |

Two nitrocellulose membranes for Western blotting were prepared as described above. After Western transfer, the gels were stained silver using a 'Sil Best Kit' (Nakarai Tesuku Kabushiki-Kaisha).

One of the two Western blotting nitrocellulose membranes prepared as above was employed for H chain (μ chain) detection and the other for L chain (K chain) detection. The following procedures were used.

After the Western transfer, the nitrocellulose membranes were immersed in an aqueous solution containing 3% (w/v) gelatin (Bio-Rad), 20 mM tris-hydrochloric acid (pH 7.5) and 500 mM of sodium chloride. Membranes were shaken gently at room temperature for one hour (the procedure described since "After Western transfer" is referred to herein as "blocking"). The thus blocked nitrocellulose membranes were immersed in a diluted antibody solution and shaken gently at room temperature for 2 hours. The resulting antibody solutions were prepared by diluting either 2.5 μl of peroxidase-labelled goat anti-mouse IgM antibody (μ chain-specific : Jackson Immuno Research Laboratories) or 25 μl of peroxidase-labelled rat anti-mouse κ chain monoclonal antibody (Zymed Laboratories) with 10 ml of buffer [20 mM tris-hydrochloric acid (pH 7.5), 500 mM sodium chloride] hereinafter referred to as "TBS".

Each nitrocellulose membrane was then immersed in 20 ml of TTBS, which is TBS containing 2% (v/v) Tween 20 (Bio-Rad) and washed by shaking gently at room temperature for 15 minutes. The treated membranes were washed with 20 ml of TTBS, which was then drained away. Residual peroxidase activity on this nitrocellulose membrane was detected using an ECL Western Blotting Detection system (Amersham). More specifically, the substrate in this system emits light during a chemical reaction under the catalytic action of peroxidase. The light emission may be detected on photographic film using an ECL mini camera (Amersham) and instant film (Type 667; Polaroid). The pictures taken were compared with the silver-stained gels to identify the protein bands that were specifically bound to the antibodies.

Antibodies to the mouse M chain cross-reacted with a protein band of approximately 78,000 Da. A band of this size was present in the supernatant of COS-1 cell samples that had been transfected with pHEX126, either singly or with another plasmid. These samples corresponded to [B], [E] and [F] of Example 5.

Antibodies to the mouse K chain cross-reacted with a protein band of approximately 26,000 Da. A band of this size was present in the supernatant of COS-1 cell samples that had been transfected with pLEX004, either singly or with another plasmid. These samples corresponded to [C], [E] and [F] of Example 5.

The antibody produced by the hybridoma was analysed by Western blotting using the same methodology. Bands corresponding to the H and L chain of CH11 from the hybridoma were seen to be the same size as those obtained from the transfected COS-1 cells.

**(6-2) Confirmation of Fas binding ability by ELISA**

(a) Preparation of antigen

The expression vector pME18S-mfas-AIC [see Nishimura, Y., *et al.,* (1995), Mol. Immunol., *154,* 4395-4403] encodes a fusion protein consisting of the extracellular region of mouse Fas antigen and the extracellular region of mouse interleukin 3 receptor. The portion of the vector encoding the extracellular region of mouse Fas antigen was replaced with a DNA fragment encoding the extracellular region of human Fas antigen. This new expression vector, phFas-AIC2, encoding a fusion protein consisting of the extracellular region of the human Fas antigen and the extracellular region of the mouse interleukin 3 receptor, was prepared as described below.

Oligonucleotide primers were designed and synthesised in order to allow PCR amplification of DNA coding for the Fas antigen extracellular region. The DNA template used was the plasmid vector pCEV4 [Itoh, N., *et al.,* (1991), Cell, *66*, 233-243] which contains the cDNA coding for the human Fas antigen. The sequences of the oligonucleotides selected were as follows:

5'-GGGGAATTCC AGTACGGAGT TGGGGAAGCT CTTT-3' (N1; SEQ ID NO. 77 of the sequence listing); and

5'-GTTTCTTCTG CCTCTGTCAC CAAGTTAGAT CTGGA-3' (C3N; SEQ ID NO. 78 of the sequence listing).

Oligonucleotide primers were also designed and synthesised in order to allow PCR amplification of the DNA coding for the extracellular region of the mouse interleukin 3 receptor. The DNA template used was the plasmid vector pME18S-mFas-AIC (Nishimara, Y. *et al.,* 1995, Mol. Immunology, 154, 4395-4403). The sequences of the oligonucleotides were as follows:

5'-TCCAGATCTA ACTTGGTGAC AGAGGCAGAA GAAAC-3' (N3N; SEQ ID NO. 79 of the sequence listing); and

5'-CCCTCTAGAC GCGTCACGTG GGCATCAC-3' (CTN2; SEQ ID NO. 80 of the sequence listing).

The PCR reaction was carried out using the above plasmids, the oligonucleotide primers and the LA PCR kit (Takara Shuzo Co., Ltd.) under the following conditions.

Composition of reaction mixture:

| | |
|---|---|
| Plasmid DNA | 20 ng |
| Oligonucleotide primers | 0.5 μg (each) |
| 10-Fold concentration LA PCA buffer | 25 μl |
| dNTP mix | 25 μl |
| Taq polymerase | 12.5 units |

Distilled water was added to make up the final volume to 250 μl; the buffer, dNTP mix and Taq polymerase were included in the kit.

Temperature conditions:

The sample was initially heated at 94°C for 2 minutes. The reaction mixture was then subjected to a heat cycle of 94°C for one minute, 55°C for one minute and 72°C for 2 minutes, and this cycle was repeated 30 times, followed by heating at 72°C for a further 10 minutes.

The products of each PCR reaction were separated by electrophoresis on an 8% polyacrylamide gel (run in TBE buffer, at 100V for 2 hours). A band of approximately 460 bp was excised from the gel, corresponding to the DNA fragment containing the gene coding for the extracellular region of the human Fas antigen. A band of approximately 1300 bp was also excised from the gel, corresponding to the DNA fragment containing the gene coding for the extracellular region of the mouse interleukin 3 receptor. DNA fragments were eluted from the gel as described in Example 5.

The two DNA fragments obtained after the first PCR reaction were used together in a second PCR reaction, along with the oligonucleotide primers C3N and N3N. Each primer hybridises to one of the original fragments. When used in a PCR reaction, the primers amplify a fragment of DNA in which two polypeptide-coding genes are ligated in the same reading frame. The second PCR reaction was carried out under the following conditions:

Composition of reaction mixture:

| | |
|---|---|
| Products from the First-run PCR | Entire volume of each |
| Oligonucleotide primers | 0.1 μg (each) |

(continued)

| | |
|---|---|
| 10-Fold concentration LA PCA buffer | 25 μl |
| dNTP mix | 25 μl |
| Taq polymerase | 12.5 units |

Distilled water was added to make up the final volume to 250 μl.

The heat cycling conditions were identical to those used in the immediately previous PCR reaction.

A portion of the reaction mixture of the second PCR was loaded and run on an agarose gel, which confirmed that the desired PCR product of approximately 1700 bp had been amplified. The remainder of the reaction mixture was then precipitated using ethanol. The precipitated DNA was digested with the restriction enzymes EcoR1 and Xba1 and the digestion products were separated by agarose gel electrophoresis on a 1% (w/v) agarose gel. A band of approximately 1700 bp was excised from the gel, and the DNA was purified from the gel in a similar manner to that described in Example 5.

In parallel, 2 μg of pME18S-mfas-AIC DNA were digested with the restriction enzymes EcoR1 and Xba1. The products of the digestion were separated by gel electrophoresis on a 0.8% (w/v) agarose gel, and a band of approximately 3000 bp was excised from the gel. The DNA was isolated and ligated to the 1700 bp PCR product (prepared above) using a DNA ligation kit version 2 (Takara Shuzo Co., Ltd.). The total reaction mixture (10 μl) was used to transform JM109 *E. coli* competent cells (Takara Shuzo) and a selection was made for ampicillin-resistant colonies. One such ampicillin resistant transformant was cultured by conventional procedures, and plasmid DNA prepared from the cells by alkaline lysis (Maniatis, *supra).* The resulting plasmid was designated phFas-AIC2.

phFAS-AIC2 DNA was transfected into COS-1 cells by electroporation, using the GTE-1 apparatus. More specifically, the COS-1 cells were cultured in four 225 $cm^2$ culturing flasks (Sumitomo Bakelite). The cells were grown to a semiconfluent state in DMEM containing 10% (v/v) foetal bovine serum (CSL). The medium was removed and the cells were treated twice with 3 ml of trypsin-EDTA solution (Sigma) at 37°C for 3 minutes. The cells were recovered, then washed twice with a PBS (-) buffer (Nissui Pharmaceutical). The washed COS-1 cells were adjusted to a density of 6 x $10^7$ cells/ml with PBS (-) buffer.

phFas-AIC2 plasmid DNA was prepared using a plasmid mass preparation kit (Maxiprep DNA Purification System; Promega) and 200 μg of this plasmid DNA was precipitated by ethanol, then dissolved in 200 μl of PBS(-) buffer.

A portion of the cell suspension (20 μl; 1.2 x $10^6$ cells) and 20 μl of plasmid solution were mixed, and the resulting mixture was introduced to an electroporation cuvette having an electrode interval of 2 mm. The cuvette was placed into the electroporation apparatus and two 600V-30μsec pulses were applied to the sample, with an interval of 1 second between pulses. This procedure was carried out on ten samples.

The cell-DNA mixtures of the 10 samples were combined and added to 50 ml of DMEM containing 10% (v/v) of foetal bovine serum and the resulting mixture was transferred into a 225 $cm^2$ cell culturing flask (Sumitomo Bakelite). The cells were cultured at 37°C under 7.5% $CO_2$ for 24 hours. After this time, the culture supernatant was removed and the cells were washed with serum-free DMEM medium. Fresh serum-free DMEM medium (50 ml) was then added to the culture, and the cells were cultured at 37°C under 7.5% $CO_2$ for a further 24 hours. After this time the culture supernatant was recovered.

(b) ELISA

Aliquots of the COS-1 culture supernatants (100 μl) were pipetted into wells of a 96-well immunoplate (Maxisorb; Nunk) and stored at 4°C overnight. This results in the formation of a solid phase of protein on the inner surface of the well, containing the extracellular region of human Fas antigen. Each well was washed with PBS containing 0.05% (v/v) Tween-20 (EIA grade, Bio-Rad), then 100 μl of PBS containing 0.5% (w/v) of bovine serum albumin (BSA) was added. The plate was left at room temperature for one hour to effect blocking.

Each well was washed with PBS containing 0.05% (v/v) Tween 20, and then a CH11 (standard) prepared in Example 2, along with 100 μl of diluted test sample solutions prepared in Example 5 were pipetted into the wells. The reaction was allowed to continue at room temperature for 4 hours.

The wells were then washed with PBS containing 0.05% (v/v) Tween-20. A portion (100 μl) of peroxidase-labelled rat anti-mouse IgM monoclonal antibody (1/2000 dilution, Zymed Laboratories) was pipetted into each well and the reaction allowed to proceed at room temperature for 2 hours.

The wells were then further washed with PBS containing 0.05% (v/v) Tween-20. A portion (100 μl) of substrate solution (Peroxidase substrate kit ABTS; Bio-Rad) was pipetted into each well to initiate a colour reaction. The absorbency values of each well at 405 nm and 492 nm were measured using a microplate reader and the absorbance reading at 492nm was subtracted from the absorbance reading at 405nm. The values thus obtained were compared to those of the CH11 standard, to evaluate the ability of the test sample to bind to the protein containing the extracellular region of human Fas antigen.

The results are shown in Table 1 below. As can be seen, binding was observed using supernatants [E] (pHEX126 and pLEX004) and [F] (pHEX126, pLEX004 and pJEX004), prepared in Example 5.

Table 1

| Sample | $OD_{405}$ - $OD_{492}$ |
|---|---|
| CH11 Standard | 0.445 |
| PBS | 0.104 |
| pHEX126 | 0.109 |
| pLEX004 | 0.135 |
| pJEX 004 | 0.107 |
| pHEX126 + pLEX004 | 0.330 |
| pHEX126 + pLEX004 + PJEX004 | 0.202 |

**EXAMPLE 7**

**Cytotoxicity Assay**

The cytotoxicity of each COS-1 cell culture supernatant prepared in Example 5 was determined by the MTT [3-(4, 5-dimethylthiazol-2-yl)-2,5-diphenyl 2H-tetrazolium bromide] assay method, described below.

The human lymphocyte cell strain HPB-ALL [see Morikawa, S., *et al.,* (1978), Int. J. Cancer, *21*, 166-170] was cultured on a RPMI1640 medium (Nissui Pharmaceutical) containing 20 mM HEPES (HEPES: N-2-Hydroxyethylpiperazine-N'-2-Ethane Sulphonic Acid), 50 μM 2-mercaptoethanol and 10% (v/v) foetal bovine serum (Equitec Bio) under 5% $CO_2$ at 37°C for 3 days. A portion (75μl) of COS-1 cell culture supernatants prepared in Example 5 were pipetted into wells of 96-well culturing plate and then 25 μl of HPB-ALL cells (adjusted to 2 x $10^5$ cells/ml) were added. The cells were cultured at 37°C in the presence of 5% $CO_2$ for 12 hours. To each well of the dish was added 10 μl of a 5mg/ml aqueous solution of MTT (Sigma) and the plate was incubated at 37°C for 4 hours. After the incubation 100 μl of a solution containing 50% (v/v) N,N-dimethylformamide and 20% (w/v) SDS was added to each well. The absorbency values for each well were read at 590 nm and 620 nm using a microplate reader (Nippon Intermed Ltd.). The absorbance at 620 nm was subtracted from the absorbance at 590 nm. The higher the value, the larger the number of viable cells remaining in the well. The resulting values are given in Table 2 below.

Table 2

| Sample | $OD_{590}$ - $OD_{620}$ |
|---|---|
| PBS | 0.864 |
| pME 18S | 0.881 |
| pHEX126 (H-chain) | 0.876 |
| pLEX004 (L-chain) | 0.887 |
| pJEX004 (J-chain) | 0.883 |
| pHEX126 + pLEX004 | 0.176 |
| pHEX126 + pLEX004 + pJEX004 | 0.242 |

Those COS-1 cell cultures that were transfected with a single plasmid in Example 5 ([A], [B], [C] and [D]) all showed high values, comparable to that of the non-transfected cells. Those cells co-transfected with pHEX126 and pLEX004 ([E] and [F]) showed low values comparable to that of a blank in which no cells were present. From these results, it was found that a cytotoxic substance is produced by co-expression of the DNA coding for the CH11 H chain and CH11 L chain.

## EXAMPLE 8

### Comparative Antibody Cytotoxicity ($ED_{50}$ Test)

Cytotoxicity of the antibodies of the present invention was evaluated by an $ED_{50}$ test. As used herein, $ED_{50}$ is the concentration of antibody at which 50% of a sample of cells expressing the Fas antigen were killed. Prior to measuring $ED_{50}$, it was necessary to accurately calculate the concentration of the antibodies under test.

### 8-1 Determination of Antibody Concentration

Into each well of a 96 well ELISA plate (NUNC) was pipetted 100 µl of an anti-mouse IgM antibody solution (0.5 µg/ml in 50 mM $Na_2HCO_3$, pH 9.6, purchased from BIOSYS), and the plate was incubated overnight at 4°C. The antibody solution was then discarded and each well was washed four times with PBS containing 0.5% Tween-20 (hereafter referred to as 'PBS-T').

'Super-Block' Blocking Buffer (Pierce) was made up in PBS, in accordance with the manufacturer's instructions and 100 µl of this buffer was pipetted into each well. The plate was then further incubated for four hours at 37°C. After this time, the blocking solution was discarded, and each well was washed four times with PBS-T.

A 100 µl sample of each antibody preparation to be tested was added to each well of the plate. The test samples comprised CH11, and supernatants [E] and [F] of Example 5. Various standard preparations comprising mouse IgM (Zymed) diluted to different concentrations were used. After the samples had been added to the wells, the plate was incubated at 37°C for 4 hours. After this time, the test and standard solutions were discarded, and each well was washed four times with PBS-T.

A sample of horseradish peroxidase-conjugated anti mouse IgM (100 µl of a 1/1000 dilution in PBS) was added to each well. This solution was then discarded and each well was washed with PBS-T four times.

A 100 µl sample prepared from a Horseradish Peroxidase Substrate Kit (Biorad) was added to each well and the plate was incubated at room temperature for 30 minutes. After this time, absorbance readings were taken at both 405 and 492 nm using a Microplate reader. The absorbance at 492 nm was then subtracted from the reading at 405 nm.

By using the absorbance values for the standard samples, it was possible to construct a standard curve from which the concentration of each test antibody was able to be determined.

It was then possible to evaluate $ED_{50}$.

### (8-2) $ED_{50}$ Calculation

The three antibody solutions, of CH11, supernatant [E] (containing H and L chains) and supernatant [F] (containing H, L and J chains) of Example 5, respectively, were incubated with HBP-ALL cells. An MTT assay, described in Example 7, was used to assess how many cells were killed by each antibody solution.

The MTT results for each antibody were calculated as a function of protein concentration obtained in (8-1) above, to determine the $ED_{50}$ value, taking the MTT as the negative control and the results for HBP-ALL cultured in the absence of antibody as the positive control. Results are shown in Table 3.

Table 3

| Antibody | $ED_{50}$ (ng/ml) |
|---|---|
| CH11 | 21.2 |
| Supernatant [E] (pHEX126 and pLEX004) | 4.5 |
| Supernatant [F] (pHEX126, pLEX004 and pJEX004) | 22.9 |

The above results clearly show that the product contained in supernatant [E] surprisingly possesses approximately 5 times more activity than either CH11 or the product contained in supernatant [F]. It seems likely that an IgM structure is formed by the product contained in supernatant [F], while the product contained in supernatant [E] forms simple antibody pairings and that, under these circumstances, $ED_{50}$ is significantly enhanced.

## Claims

1. A recombinant protein, the protein comprising at least one region corresponding to an immunoglobulin variable region, wherein the at least one region enables the protein to recognize and specifically bind to an antigen, the antigen being human Fas, and wherein the protein has substantially no more immunogenicity in a human patient than a human antibody.

2. A protein according to claim 1, which consists essentially of only one variable region.

3. A protein according to claim 1, which consists essentially of two variable regions, one of the variable regions corresponding to an H chain variable region and the other to an L chain variable region.

4. A protein according to claim 1 or 3, wherein the variable regions are connected by a flexible peptide linkage.

5. A protein according to claim 1, 3 or 4, having at least two of the variable regions, the variable regions being derived from a single monoclonal antibody.

6. A protein according to claim 5, wherein the variable regions are integrally bound with human constant regions.

7. A protein according to claim 5 or 6, wherein the variable regions correspond to either an H chain variable region or to an L chain variable region, and are individually bound to an H chain constant region or an L chain constant region, respectively.

8. A protein according to any of claims 5 to 7, wherein the variable regions are each respectively a part of a larger polypeptide and the two polypeptides associate to form an antibody heterodimer.

9. A protein according to any of claims 5 to 8, wherein the variable regions are either identical with or correspond closely to those found in CH11.

10. A genetically engineered immunoglobulin protein which specifically binds to human Fas, wherein the immunoglobulin protein consists essentially of an H chain subunit and an L chain subunit, the H chain subunit comprising an amino acid sequence represented by the following general formula (I):

$$-FRH_1-CDRH_1-FRH_2-CDRH_2-FRH_3-CDRH_3-FRH_4- \quad (I)$$

wherein $FRH_1$ represents an amino acid sequence comprising 13 to 30 amino acid residues, $CDRH_1$ represents the amino acid sequence of SEQ ID NO. 1, $FRH_2$ represents an amino acid sequence comprising 14 amino acid residues, $CDRH_2$ represents the amino acid sequence of SEQ ID NO. 2, $FRH_3$ represents an amino acid sequence comprising 32 amino acid residues, $CDRH_3$ represents the amino acid sequence of SEQ ID NO. 3, and $FRH_4$ represents an amino acid sequence comprising 11 amino acid residues, each of the amino acid sequences being linked to the next *via* a peptide bond;
said L chain subunit comprising an amino acid sequence represented by the following general formula (II):

$$-FRL_1-CDRL_1-FRL_2-CDRL_2-FRL_3-CDRL_3-FRL_4- \quad (II)$$

wherein $FRL_1$ represents an amino acid sequence comprising 23 amino acid residues, $CDRL_1$ represents the amino acid sequence of SEQ ID NO. 4, $FRL_2$ represents an amino acid sequence comprising 15 amino acid residues, $CDRL_2$ represents the amino acid sequence of SEQ ID NO. 5, $FRL_3$ represents an amino acid sequence comprising 32 amino acid residues, $CDRL_3$ represents the amino acid sequence of SEQ ID NO. 6, and $FRL_4$ represents an amino acid sequence comprising 10 amino acid residues, each of the amino acid sequences being linked to the next *via* a peptide bond.

**11.** A protein according to claim 10, when the H chain subunit comprises the amino acid sequence represented by amino acids No. 1 to 116 in SEQ ID NO. 8.

**12.** A protein according to claim 10 or 11, when the L chain subunit comprises the amino acid sequence represented by amino acids No. 1 to 112 in SEQ ID NO. 10.

**13.** A protein according to claim 11 or 12, wherein the H chain subunit comprises the amino acid sequence represented by amino acids No. 1 to 571 in SEQ ID NO. 8.

**14.** A protein according to claim 11, 12 or 13, wherein the L chain subunit comprises the amino acid sequence represented by amino acids No. 1 to 219 in SEQ ID NO. 10.

**15.** RNA encoding a protein according to any preceding claim.

**16.** DNA encoding a protein according to any of claims 1 to 14.

**17.** DNA encoding a peptide of formula (I) as defined in claim 10.

**18.** DNA according to claim 17, which comprises the nucleotide sequence represented by nucleotides No. 58 to 405 in SEQ ID NO. 7.

**19.** DNA which hybridizes with DNA according to claim 18, a sense strand corresponding to the hybridizing DNA encoding an immunoglobulin H chain subunit capable of specifically binding human Fas together with an L chain subunit comprising an amino acid sequence of general formula (II) as defined in claim 10.

**20.** Hybridizing DNA according to claim 19 which hybridizes at 60 to 70°C and in 6 x SSC.

**21.** DNA coding for an L chain subunit comprising an amino acid sequence represented by general formula (II) as defined in claim 10.

**22.** DNA according to claim 21, which comprises the nucleotide sequence represented by nucleotides No. 58 to 393 in SEQ ID NO. 9.

**23.** DNA which hybridizes with DNA according to claim 22, a sense strand corresponding to the hybridizing DNA encoding an immunoglobulin L chain subunit capable of specifically binding human Fas together with an H chain subunit comprising an amino acid sequence of general formula (I) as defined in claim 10.

**24.** Hybridizing DNA according to claim 23 which hybridizes at 60 to 70°C and in 6 x SSC.

**25.** A vector comprising DNA according to any of claims 16 to 24.

**26.** A vector according to claim 25 which is an expression vector.

**27.** A vector selected from pCR3-H123 and pCR3-L103.

**28.** A host cell transformed with an expression vector according to any of claims 25 to 27.

**29.** *E. coli* pCR3-H123 (FERM BP-5247).

**30.** *E. coli* pCR3-L103 (FERM BP-5248).

**31.** A method for producing an immunoglobulin protein which specifically recognizes human Fas antigen comprising culturing a cell according to any of claims 28 to 30 under conditions which enable expression of DNA encoding the immunoglobulin H chain or L chain subunit contained in the vector, and recovering the immunoglobulin protein from the culture.

**32.** The use of CDR's selected from $CDRH_1$, $CDRH_2$, $CDRH_3$, $CDRL_1$, $CDRL_2$ and $CDRL_3$, as defined in claim 10, and combinations thereof, in the construction of humanized antibodies.

**33.** The use of DNA encoding CDR's selected from $CDRH_1$, $CDRH_2$, $CDRH_3$, $CDRL_1$, $CDRL_2$ and $CDRL_3$, as defined in claim 10, and combinations thereof, in the construction of DNA encoding humanized antibodies.

**34.** Use of a protein according to any of claims 1 to 14 in the preparation of a medicament for the treatment of an autoimmune disease.

**35.** Use according to claim 34, wherein the autoimmune disease is rheumatism.

FIG 1.

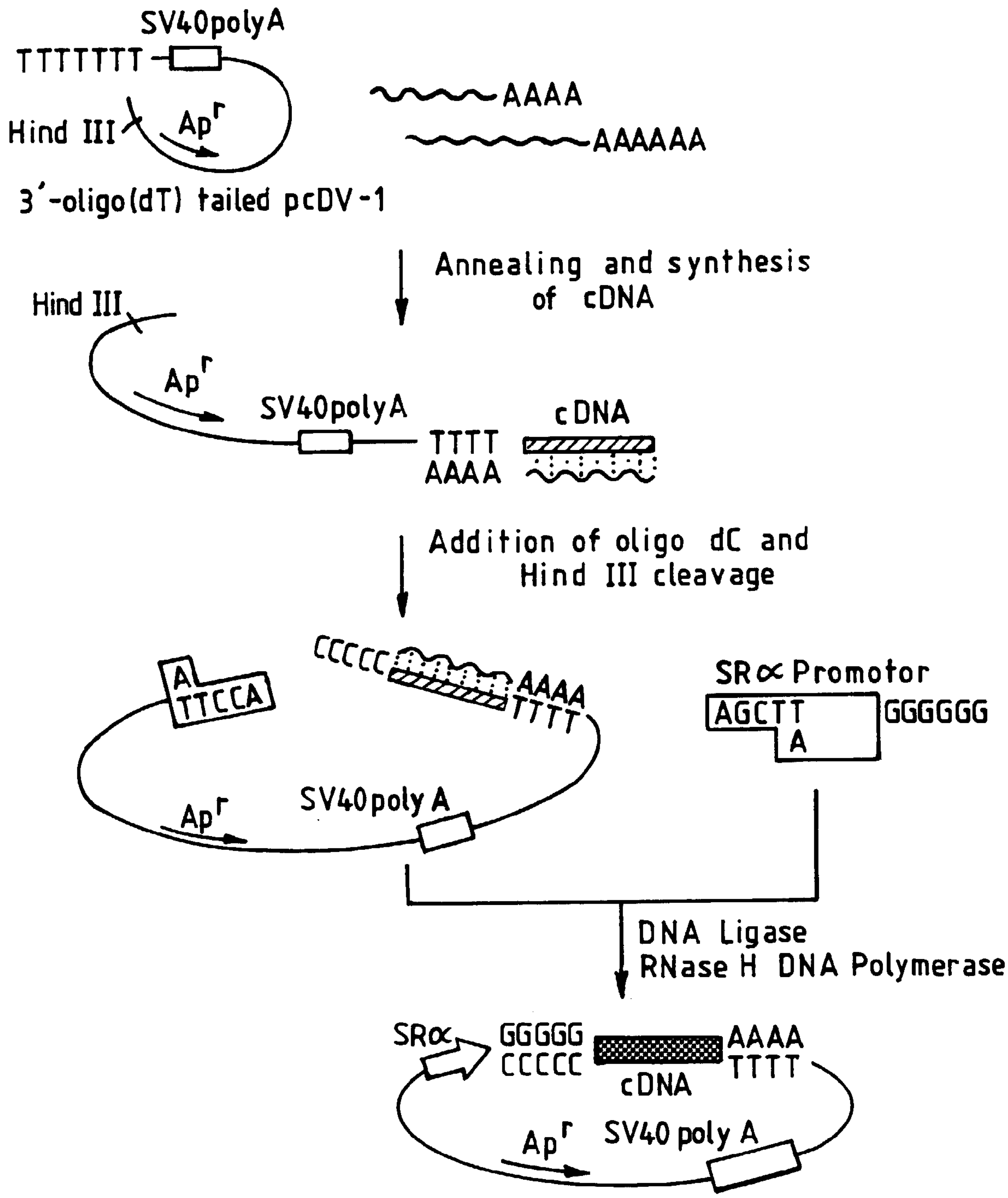
SV40polyA
TTTTTTT
Hind III
Apr
AAAA
AAAAAA
3'-oligo(dT) tailed pcDV-1
Annealing and synthesis of cDNA
Hind III
Apr
SV40polyA
cDNA
TTTT
AAAA
Addition of oligo dC and Hind III cleavage
A
TTCCA
CCCCC
AAAA
TTTT
SRα Promotor
AGCTT
A
GGGGGG
Apr
SV40poly A
DNA Ligase
RNase H DNA Polymerase
SRα
GGGGG
CCCCC
AAAA
TTTT
cDNA
Apr
SV40 poly A

FIG. 2

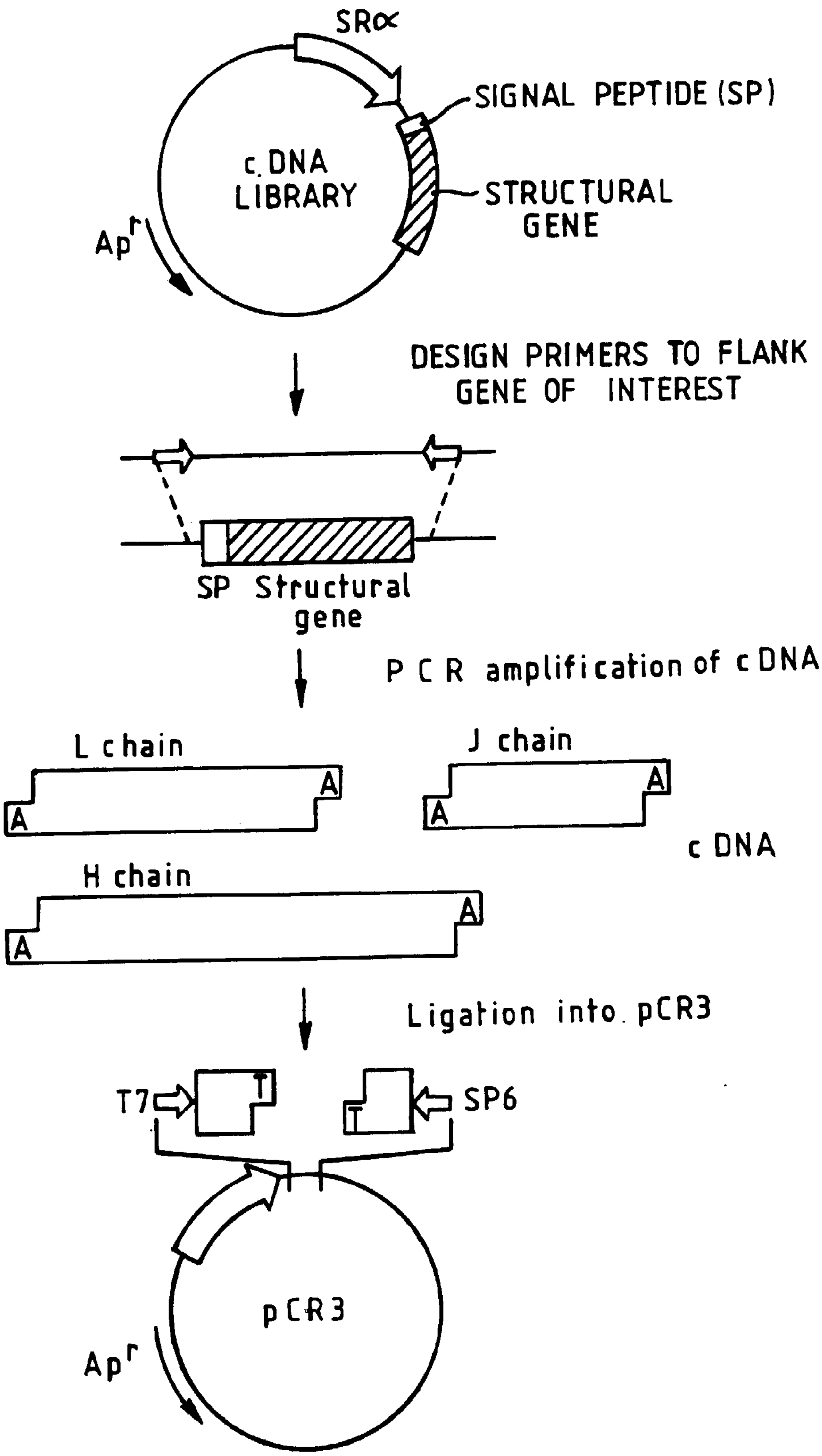
SRα
SIGNAL PEPTIDE (SP)
c.DNA LIBRARY
STRUCTURAL GENE
Ap^r
DESIGN PRIMERS TO FLANK GENE OF INTEREST
SP Structural gene
PCR amplification of cDNA
L chain
J chain
H chain
A
cDNA
Ligation into pCR3
T7
SP6
T
pCR3
Ap^r

European Patent Office

# EUROPEAN SEARCH REPORT

Application Number
EP 97 30 2415

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO 95 10540 A (IMMUNEX CORP.) 20 April 1995<br>* page 6, line 7 - page 8, line 8 *<br>* claims *<br>--- | 1-14, 32-35 | C12N15/13<br>C07K16/28<br>C12N15/70<br>C12N1/21<br>A61K39/395 |
| D,A | INTERNATIONAL IMMUNOLOGY,<br>vol. 6, no. 12, December 1994, OXFORD, GB,<br>pages 1849-1856, XP000676464<br>S. YONEHARA ET AL.: "Involvement of apoptosis antigen Fas in clonal deletion of human thymocytes."<br>* Methods *<br>--- | 1-14 | |
| D,A | CELL,<br>vol. 66, no. 2, 26 July 1991, CAMBRIDGE, MA, USA,<br>pages 233-243, XP000579691<br>N. ITOH ET AL.: "The polypeptide encoded by the cDNA for human cell surface antigen Fas can mediate apoptosis."<br>* the whole document *<br>--- | 1-14 | TECHNICAL FIELDS SEARCHED (Int.Cl.6) |
| A | ARTHRITIS AND RHEUMATISM,<br>vol. 37, no. 9 suppl., September 1994, NEW YORK, NY, USA,<br>page S311 XP000566308<br>B. HEILIG ET AL.: "Anti-CD95 mAb induces apoptosis in synovial CD45RO+ cells of rheumatoid arthritis patients."<br>* abstract 901 *<br>--- | 1-14, 32-35 | C07K |
| P,X | WO 96 40041 A (CHIRON CORP.) 19 December 1996<br>* page 4, line 13 - line 21 *<br>* claims *<br>----- | 1-14, 32-35 | |

The present search report has been drawn up for all claims

| Place of search | Date of completion of the search | Examiner |
|---|---|---|
| THE HAGUE | 23 June 1997 | Nooij, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

SEQUENCE LISTING

(1) GENERAL INFORMATION:

(i) APPLICANT:
(A) NAME: Sankyo Company, Limited
(B) STREET: 5-1, Nihonbashi Honcho 3-chome, Chuo-ku
(C) CITY: Tokyo
(E) COUNTRY: Japan
(F) POSTAL CODE (ZIP): 103
(G) TELEPHONE: 81-3-5255-7111

(ii) TITLE OF INVENTION: Recombinant Anti Human Fas Antibody

(iii) NUMBER OF SEQUENCES: 80

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: JP Hei 8-78570
(B) FILING DATE: 01-APR-1996

(2) INFORMATION FOR SEQ ID NO: 1:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 1:

```
Asp Tyr Asn Met His
1               5
```

(2) INFORMATION FOR SEQ ID NO: 2:

(i) SEQUENCE CHARACTERISTICS:
(A) LENGTH: 17 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 2:

Tyr Ile Tyr Pro Tyr Asn Gly Gly Thr Gly Tyr Asn Gln Lys Phe Lys
1 5 10 15

Ser

(2) INFORMATION FOR SEQ ID NO: 3:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 3:

Ser Tyr Tyr Ala Met Asp Tyr
1 5

(2) INFORMATION FOR SEQ ID NO: 4:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 4:

Arg Ser Ser Lys Ser Leu Val His Ser Asn Gly Asn Thr Tyr Leu His
1 5 10 15

(2) INFORMATION FOR SEQ ID NO: 5:

(i) SEQUENCE CHARACTERISTICS:
(A) LENGTH: 7 amino acids

(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 5:

```
Lys Val Ser Asn Arg Phe Ser
1               5
```

(2) INFORMATION FOR SEQ ID NO: 6:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 6:

```
Ser Gln Ser Thr His Val Pro Pro Ala
1               5
```

(2) INFORMATION FOR SEQ ID NO: 7:

(i) SEQUENCE CHARACTERISTICS:
(A) LENGTH: 1773 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Mus musculus
(G) CELL TYPE: Hybridoma
(H) CELL LINE: CH11

(ix) FEATURE:
(A) NAME/KEY: CDS
(B) LOCATION:1..1770

(ix) FEATURE:
(A) NAME/KEY: mat_peptide
(B) LOCATION:58..1770

(ix) FEATURE:
(A) NAME/KEY: sig_peptide
(B) LOCATION:1..57

(xi) SEQUENCE DESCRIPTION: SEQ ID NO: 7:

```
ATG GGA TGG AGC TGG ATC TTT CTC TTC CTC CTG TCA GGA ACT GCA GGC         48
Met Gly Trp Ser Trp Ile Phe Leu Phe Leu Leu Ser Gly Thr Ala Gly
-19             -15                 -10                  -5

GTC CAC TCT GAG GTC CAG CTT CAG CAG TCA GGA CCT GAG CTG GTG AAA         96
Val His Ser Glu Val Gln Leu Gln Gln Ser Gly Pro Glu Leu Val Lys
             1               5                  10

CCT GGG GCC TCA GTG AAG ATA TCC TGC AAG GCT TCT GGA TAC ACA TTC        144
Pro Gly Ala Ser Val Lys Ile Ser Cys Lys Ala Ser Gly Tyr Thr Phe
     15                  20                  25

ACT GAC TAC AAC ATG CAC TGG GTG AAG CAG AGC CAT GGA AAG AGC CTT        192
Thr Asp Tyr Asn Met His Trp Val Lys Gln Ser His Gly Lys Ser Leu
 30                  35                  40                  45

GAG TGG ATT GGA TAT ATT TAT CCT TAC AAT GGT GGT ACT GGC TAC AAC        240
Glu Trp Ile Gly Tyr Ile Tyr Pro Tyr Asn Gly Gly Thr Gly Tyr Asn
                 50                  55                  60

CAG AAG TTC AAG AGC AAG GCC ACA TTG ACT GTT GAC AAT TCC TCC AGC        288
Gln Lys Phe Lys Ser Lys Ala Thr Leu Thr Val Asp Asn Ser Ser Ser
             65                  70                  75

ACA GCC TAC ATG GAG CTC CGC AGC CTG ACA TCT GAG GAC TCT GCA GTC        336
Thr Ala Tyr Met Glu Leu Arg Ser Leu Thr Ser Glu Asp Ser Ala Val
         80                  85                  90

TAT TAC TGT GCA AGA AGT TAC TAT GCT ATG GAC TAC TGG GGT CAA GGA        384
Tyr Tyr Cys Ala Arg Ser Tyr Tyr Ala Met Asp Tyr Trp Gly Gln Gly
     95                 100                 105

ACC TCA GTC ACC GTC TCC TCA GAG AGT CAG TCC TTC CCA AAT GTC TTC        432
Thr Ser Val Thr Val Ser Ser Glu Ser Gln Ser Phe Pro Asn Val Phe
110                 115                 120                 125

CCC CTC GTC TCC TGC GAG AGC CCC CTG TCT GAT AAG AAT CTG GTG GCC        480
Pro Leu Val Ser Cys Glu Ser Pro Leu Ser Asp Lys Asn Leu Val Ala
                130                 135                 140

ATG GGC TGC CTA GCC CGG GAC TTC CTG CCC AGC ACC ATT TCC TTC ACC        528
Met Gly Cys Leu Ala Arg Asp Phe Leu Pro Ser Thr Ile Ser Phe Thr
            145                 150                 155

TGG AAC TAC CAG AAC AAC ACT GAA GTC ATC CAG GGT ATC AGA ACC TTC        576
Trp Asn Tyr Gln Asn Asn Thr Glu Val Ile Gln Gly Ile Arg Thr Phe
```

```
         160                 165                 170

CCA ACA CTG AGG ACA GGG GGC AAG TAC CTA GCC ACC TCG CAG GTG TTG        624
Pro Thr Leu Arg Thr Gly Gly Lys Tyr Leu Ala Thr Ser Gln Val Leu
    175                 180                 185

CTG TCT CCC AAG AGC ATC CTT GAA GGT TCA GAT GAA TAC CTG GTA TGC        672
Leu Ser Pro Lys Ser Ile Leu Glu Gly Ser Asp Glu Tyr Leu Val Cys
190                 195                 200                 205

AAA ATC CAC TAC GGA GGC AAA AAC AGA GAT CTG CAT GTG CCC ATT CCA        720
Lys Ile His Tyr Gly Gly Lys Asn Arg Asp Leu His Val Pro Ile Pro
                210                 215                 220

GCT GTC GCA GAG ATG AAC CCC AAT GTA AAT GTG TTC GTC CCA CCA CGG        768
Ala Val Ala Glu Met Asn Pro Asn Val Asn Val Phe Val Pro Pro Arg
            225                 230                 235

GAT GGC TTC TCT GGC CCT GCA CCA CGC AAG TCT AAA CTC ATC TGC GAG        816
Asp Gly Phe Ser Gly Pro Ala Pro Arg Lys Ser Lys Leu Ile Cys Glu
        240                 245                 250

GCC ACG AAC TTC ACT CCA AAA CCG ATC ACA GTA TCC TGG CTA AAG GAT        864
Ala Thr Asn Phe Thr Pro Lys Pro Ile Thr Val Ser Trp Leu Lys Asp
    255                 260                 265

GGG AAG CTC GTG GAA TCT GGC TTC ACC ACA GAT CCG GTG ACC ATC GAG        912
Gly Lys Leu Val Glu Ser Gly Phe Thr Thr Asp Pro Val Thr Ile Glu
270                 275                 280                 285

AAC AAA GGA TCC ACA CCC CAA ACC TAC AAG GTC ATA AGC ACA CTT ACC        960
Asn Lys Gly Ser Thr Pro Gln Thr Tyr Lys Val Ile Ser Thr Leu Thr
                290                 295                 300

ATC TCT GAA ATC GAC TGG CTG AAC CTG AAT GTG TAC ACC TGC CGT GTG       1008
Ile Ser Glu Ile Asp Trp Leu Asn Leu Asn Val Tyr Thr Cys Arg Val
            305                 310                 315

GAT CAC AGG GGT CTC ACC TTC TTG AAG AAC GTG TCC TCC ACA TGT GCT       1056
Asp His Arg Gly Leu Thr Phe Leu Lys Asn Val Ser Ser Thr Cys Ala
        320                 325                 330

GCC AGT CCC TCC ACA GAC ATC CTA ACC TTC ACC ATC CCC CCC TCC TTT       1104
Ala Ser Pro Ser Thr Asp Ile Leu Thr Phe Thr Ile Pro Pro Ser Phe
    335                 340                 345

GCC GAC ATC TTC CTC AGC AAG TCC GCT AAC CTG ACC TGT CTG GTC TCA       1152
Ala Asp Ile Phe Leu Ser Lys Ser Ala Asn Leu Thr Cys Leu Val Ser
350                 355                 360                 365

AAC CTG GCA ACC TAT GAA ACC CTG AAT ATC TCC TGG GCT TCT CAA AGT       1200
Asn Leu Ala Thr Tyr Glu Thr Leu Asn Ile Ser Trp Ala Ser Gln Ser
                370                 375                 380

GGT GAA CCA CTG GAA ACC AAA ATT AAA ATC ATG GAA AGC CAT CCC AAT       1248
Gly Glu Pro Leu Glu Thr Lys Ile Lys Ile Met Glu Ser His Pro Asn
            385                 390                 395
```

```
GGC ACC TTC AGT GCT AAG GGT GTG GCT AGT GTT TGT GTG GAA GAC TGG       1296
Gly Thr Phe Ser Ala Lys Gly Val Ala Ser Val Cys Val Glu Asp Trp
        400                 405                 410

AAT AAC AGG AAG GAA TTT GTG TGT ACT GTG ACT CAC AGG GAT CTG CCT       1344
Asn Asn Arg Lys Glu Phe Val Cys Thr Val Thr His Arg Asp Leu Pro
    415                 420                 425

TCA CCA CAG AAG AAA TTC ATC TCA AAA CCC AAT GAG GTG CAC AAA CAT       1392
Ser Pro Gln Lys Lys Phe Ile Ser Lys Pro Asn Glu Val His Lys His
430                 435                 440                 445

CCA CCT GCT GTG TAC CTG CTG CCA CCA GCT CGT GAG CAA CTG AAC CTG       1440
Pro Pro Ala Val Tyr Leu Leu Pro Pro Ala Arg Glu Gln Leu Asn Leu
                450                 455                 460

AGG GAG TCA GCC ACA GTC ACC TGC TTG GTG AAG GGC TTC TCT CCT GCA       1488
Arg Glu Ser Ala Thr Val Thr Cys Leu Val Lys Gly Phe Ser Pro Ala
            465                 470                 475

GAC ATC AGT GTG CAG TGG CTT CAG AGA GGG CAA CTC TTG CCC CAA GAG       1536
Asp Ile Ser Val Gln Trp Leu Gln Arg Gly Gln Leu Leu Pro Gln Glu
        480                 485                 490

AAG TAT GTG ACC AGT GCC CCG ATG CCA GAG CCT GGG GCC CCA GGC TTC       1584
Lys Tyr Val Thr Ser Ala Pro Met Pro Glu Pro Gly Ala Pro Gly Phe
    495                 500                 505

TAC TTT ACC CAC AGC ATC CTG ACT GTG ACA GAG GAG GAA TGG AAC TCC       1632
Tyr Phe Thr His Ser Ile Leu Thr Val Thr Glu Glu Glu Trp Asn Ser
510                 515                 520                 525

GGA GAG ACC TAT ACC TGT GTT GTA GGC CAC GAG GCC CTG CCA CAC CTG       1680
Gly Glu Thr Tyr Thr Cys Val Val Gly His Glu Ala Leu Pro His Leu
                530                 535                 540

GTG ACC GAG AGG ACC GTG GAC AAG TCC ACT GGT AAA CCC ACA CTG TAC       1728
Val Thr Glu Arg Thr Val Asp Lys Ser Thr Gly Lys Pro Thr Leu Tyr
            545                 550                 555

AAT GTC TCC CTG ATC ATG TCT GAC ACA GGC GGC ACC TGC TAT               1770
Asn Val Ser Leu Ile Met Ser Asp Thr Gly Gly Thr Cys Tyr
        560                 565                 570

TGA                                                                   1773
```

(2) INFORMATION FOR SEQ ID NO: 8:

(i) SEQUENCE CHARACTERISTICS:
(A) LENGTH: 590 amino acids
(B) TYPE: amino acid
(D) TOPOLOGY: linear

(ii) MOLECULE TYPE: protein
(xi) SEQUENCE DESCRIPTION: SEQ ID NO: 8:

```
Met Gly Trp Ser Trp Ile Phe Leu Phe Leu Leu Ser Gly Thr Ala Gly
```

```
-19                 -15                     -10                     -5

Val His Ser Glu Val Gln Leu Gln Gln Ser Gly Pro Glu Leu Val Lys
                1               5                   10

Pro Gly Ala Ser Val Lys Ile Ser Cys Lys Ala Ser Gly Tyr Thr Phe
    15                  20                  25

Thr Asp Tyr Asn Met His Trp Val Lys Gln Ser His Gly Lys Ser Leu
30                  35                  40                      45

Glu Trp Ile Gly Tyr Ile Tyr Pro Tyr Asn Gly Gly Thr Gly Tyr Asn
                50                  55                  60

Gln Lys Phe Lys Ser Lys Ala Thr Leu Thr Val Asp Asn Ser Ser Ser
            65                  70                  75

Thr Ala Tyr Met Glu Leu Arg Ser Leu Thr Ser Glu Asp Ser Ala Val
        80                  85                  90

Tyr Tyr Cys Ala Arg Ser Tyr Tyr Ala Met Asp Tyr Trp Gly Gln Gly
    95                  100                 105

Thr Ser Val Thr Val Ser Ser Glu Ser Gln Ser Phe Pro Asn Val Phe
110                 115                 120                     125

Pro Leu Val Ser Cys Glu Ser Pro Leu Ser Asp Lys Asn Leu Val Ala
                130                 135                 140

Met Gly Cys Leu Ala Arg Asp Phe Leu Pro Ser Thr Ile Ser Phe Thr
            145                 150                 155

Trp Asn Tyr Gln Asn Asn Thr Glu Val Ile Gln Gly Ile Arg Thr Phe
        160                 165                 170

Pro Thr Leu Arg Thr Gly Gly Lys Tyr Leu Ala Thr Ser Gln Val Leu
    175                 180                 185

Leu Ser Pro Lys Ser Ile Leu Glu Gly Ser Asp Glu Tyr Leu Val Cys
190                 195                 200                     205

Lys Ile His Tyr Gly Gly Lys Asn Arg Asp Leu His Val Pro Ile Pro
                210                 215                 220

Ala Val Ala Glu Met Asn Pro Asn Val Asn Val Phe Val Pro Pro Arg
            225                 230                 235

Asp Gly Phe Ser Gly Pro Ala Pro Arg Lys Ser Lys Leu Ile Cys Glu
        240                 245                 250

Ala Thr Asn Phe Thr Pro Lys Pro Ile Thr Val Ser Trp Leu Lys Asp
    255                 260                 265

Gly Lys Leu Val Glu Ser Gly Phe Thr Thr Asp Pro Val Thr Ile Glu
270                 275                 280                     285

Asn Lys Gly Ser Thr Pro Gln Thr Tyr Lys Val Ile Ser Thr Leu Thr
                290                 295                 300
```

```
Ile Ser Glu Ile Asp Trp Leu Asn Leu Asn Val Tyr Thr Cys Arg Val
            305                 310                 315

Asp His Arg Gly Leu Thr Phe Leu Lys Asn Val Ser Ser Thr Cys Ala
        320                 325                 330

Ala Ser Pro Ser Thr Asp Ile Leu Thr Phe Thr Ile Pro Pro Ser Phe
    335                 340                 345

Ala Asp Ile Phe Leu Ser Lys Ser Ala Asn Leu Thr Cys Leu Val Ser
350                 355                 360                 365

Asn Leu Ala Thr Tyr Glu Thr Leu Asn Ile Ser Trp Ala Ser Gln Ser
                370                 375                 380

Gly Glu Pro Leu Glu Thr Lys Ile Lys Ile Met Glu Ser His Pro Asn
            385                 390                 395

Gly Thr Phe Ser Ala Lys Gly Val Ala Ser Val Cys Val Glu Asp Trp
        400                 405                 410

Asn Asn Arg Lys Glu Phe Val Cys Thr Val Thr His Arg Asp Leu Pro
    415                 420                 425

Ser Pro Gln Lys Lys Phe Ile Ser Lys Pro Asn Glu Val His Lys His
430                 435                 440                 445

Pro Pro Ala Val Tyr Leu Leu Pro Pro Ala Arg Glu Gln Leu Asn Leu
                450                 455                 460

Arg Glu Ser Ala Thr Val Thr Cys Leu Val Lys Gly Phe Ser Pro Ala
            465                 470                 475

Asp Ile Ser Val Gln Trp Leu Gln Arg Gly Gln Leu Leu Pro Gln Glu
        480                 485                 490

Lys Tyr Val Thr Ser Ala Pro Met Pro Glu Pro Gly Ala Pro Gly Phe
    495                 500                 505

Tyr Phe Thr His Ser Ile Leu Thr Val Thr Glu Glu Glu Trp Asn Ser
510                 515                 520                 525

Gly Glu Thr Tyr Thr Cys Val Val Gly His Glu Ala Leu Pro His Leu
                530                 535                 540

Val Thr Glu Arg Thr Val Asp Lys Ser Thr Gly Lys Pro Thr Leu Tyr
            545                 550                 555

Asn Val Ser Leu Ile Met Ser Asp Thr Gly Gly Thr Cys Tyr
        560                 565                 570
```

(2) INFORMATION FOR SEQ ID NO: 9:

(i) SEQUENCE CHARACTERISTICS:
- (A) LENGTH: 717 base pairs
- (B) TYPE: nucleic acid
- (C) STRANDEDNESS: double

```
        (D) TOPOLOGY: linear

    (ii) MOLECULE TYPE: cDNA to mRNA

   (iii) HYPOTHETICAL: NO

    (iv) ANTI-SENSE: NO

    (vi) ORIGINAL SOURCE:
        (A) ORGANISM: Mus musculus
        (G) CELL TYPE: Hybridoma
        (H) CELL LINE: CH11

    (ix) FEATURE:
        (A) NAME/KEY: CDS
        (B) LOCATION:1..714

    (ix) FEATURE:
        (A) NAME/KEY: mat_peptide
        (B) LOCATION:58..714

    (ix) FEATURE:
        (A) NAME/KEY: sig_peptide
        (B) LOCATION:1..57


    (xi) SEQUENCE DESCRIPTION: SEQ ID NO: 9:

ATG AAG TTG CCT GTT AGG CTG TTG GTG CTG ATG TTC TGG ATT CCT GCT       48
Met Lys Leu Pro Val Arg Leu Leu Val Leu Met Phe Trp Ile Pro Ala
-19             -15                 -10                 -5

TCC AGC AGT GAT GTT GTG ATG ACC CAA AGT CCA CTC TCC CTG CCT GTC       96
Ser Ser Ser Asp Val Val Met Thr Gln Ser Pro Leu Ser Leu Pro Val
              1               5                  10

AGT CTT GGA GAT CAA GCC TCC ATC TCT TGC AGA TCT AGT AAG AGC CTT      144
Ser Leu Gly Asp Gln Ala Ser Ile Ser Cys Arg Ser Ser Lys Ser Leu
     15                  20                  25

GTA CAC AGT AAT GGA AAC ACC TAT TTA CAT TGG TAC CTG CAG AAG CCA      192
Val His Ser Asn Gly Asn Thr Tyr Leu His Trp Tyr Leu Gln Lys Pro
 30                 35                  40                  45

GGC CAG TCT CCA AAG CTC CTG ATC TAC AAA GTT TCC AAC CGA TTT TCT      240
Gly Gln Ser Pro Lys Leu Leu Ile Tyr Lys Val Ser Asn Arg Phe Ser
                 50                  55                  60

GGG GTC CCA GAC AGG TTC AGT GGC AGT GGA TCA GGG ACA GAT TTC ACA      288
Gly Val Pro Asp Arg Phe Ser Gly Ser Gly Ser Gly Thr Asp Phe Thr
             65                  70                  75

CTC AAG ATC AGC AGA GTG GAG GCT GAG GAT CTG GGA GTT TAT TTC TGC      336
Leu Lys Ile Ser Arg Val Glu Ala Glu Asp Leu Gly Val Tyr Phe Cys
         80                  85                  90

TCT CAA AGT ACA CAT GTT CCT CCG GCG TTC GGT GGA GGC ACC AAG CTG      384
Ser Gln Ser Thr His Val Pro Pro Ala Phe Gly Gly Gly Thr Lys Leu
```

```
        95                  100                 105

GAA ATC AAA CGG GCT GAT GCT GCA CCA ACT GTA TCC ATC TTC CCA CCA        432
Glu Ile Lys Arg Ala Asp Ala Ala Pro Thr Val Ser Ile Phe Pro Pro
110                 115                 120                 125

TCC AGT GAG CAG TTA ACA TCT GGA GGT GCC TCA GTC GTG TGC TTC TTG        480
Ser Ser Glu Gln Leu Thr Ser Gly Gly Ala Ser Val Val Cys Phe Leu
                130                 135                 140

AAC AAC TTC TAC CCC AAA GAC ATC AAT GTC AAG TGG AAG ATT GAT GGC        528
Asn Asn Phe Tyr Pro Lys Asp Ile Asn Val Lys Trp Lys Ile Asp Gly
            145                 150                 155

AGT GAA CGA CAA AAT GGC GTC CTG AAC AGT TGG ACT GAT CAG GAC AGC        576
Ser Glu Arg Gln Asn Gly Val Leu Asn Ser Trp Thr Asp Gln Asp Ser
        160                 165                 170

AAA GAC AGC ACC TAC AGC ATG AGC AGC ACC CTC ACG TTG ACC AAG GAC        624
Lys Asp Ser Thr Tyr Ser Met Ser Ser Thr Leu Thr Leu Thr Lys Asp
    175                 180                 185

GAG TAT GAA CGA CAT AAC AGC TAT ACC TGT GAG GCC ACT CAC AAG ACA        672
Glu Tyr Glu Arg His Asn Ser Tyr Thr Cys Glu Ala Thr His Lys Thr
190                 195                 200                 205

TCA ACT TCA CCC ATT GTC AAG AGC TTC AAC AGG AAT GAG TGT                714
Ser Thr Ser Pro Ile Val Lys Ser Phe Asn Arg Asn Glu Cys
                210                 215

TAG                                                                    717
```

(2) INFORMATION FOR SEQ ID NO: 10:

(i) SEQUENCE CHARACTERISTICS:
(A) LENGTH: 238 amino acids
(B) TYPE: amino acid
(D) TOPOLOGY: linear

(ii) MOLECULE TYPE: protein
(xi) SEQUENCE DESCRIPTION: SEQ ID NO: 10:

```
Met Lys Leu Pro Val Arg Leu Leu Val Leu Met Phe Trp Ile Pro Ala
-19             -15                 -10                 -5

Ser Ser Ser Asp Val Val Met Thr Gln Ser Pro Leu Ser Leu Pro Val
            1                   5                  10

Ser Leu Gly Asp Gln Ala Ser Ile Ser Cys Arg Ser Ser Lys Ser Leu
    15                  20                  25

Val His Ser Asn Gly Asn Thr Tyr Leu His Trp Tyr Leu Gln Lys Pro
 30                 35                  40                  45

Gly Gln Ser Pro Lys Leu Leu Ile Tyr Lys Val Ser Asn Arg Phe Ser
                50                  55                  60
```

```
Gly Val Pro Asp Arg Phe Ser Gly Ser Gly Ser Gly Thr Asp Phe Thr
            65                  70                  75

Leu Lys Ile Ser Arg Val Glu Ala Glu Asp Leu Gly Val Tyr Phe Cys
        80                  85                  90

Ser Gln Ser Thr His Val Pro Pro Ala Phe Gly Gly Gly Thr Lys Leu
    95                  100                 105

Glu Ile Lys Arg Ala Asp Ala Ala Pro Thr Val Ser Ile Phe Pro Pro
110                 115                 120                 125

Ser Ser Glu Gln Leu Thr Ser Gly Gly Ala Ser Val Val Cys Phe Leu
                130                 135                 140

Asn Asn Phe Tyr Pro Lys Asp Ile Asn Val Lys Trp Lys Ile Asp Gly
            145                 150                 155

Ser Glu Arg Gln Asn Gly Val Leu Asn Ser Trp Thr Asp Gln Asp Ser
        160                 165                 170

Lys Asp Ser Thr Tyr Ser Met Ser Ser Thr Leu Thr Leu Thr Lys Asp
    175                 180                 185

Glu Tyr Glu Arg His Asn Ser Tyr Thr Cys Glu Ala Thr His Lys Thr
190                 195                 200                 205

Ser Thr Ser Pro Ile Val Lys Ser Phe Asn Arg Asn Glu Cys
                210                 215
```

(2) INFORMATION FOR SEQ ID NO: 11:

(i) SEQUENCE CHARACTERISTICS:
- (A) LENGTH: 480 base pairs
- (B) TYPE: nucleic acid
- (C) STRANDEDNESS: double
- (D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
- (A) ORGANISM: Mus musculus
- (G) CELL TYPE: Hybridoma
- (H) CELL LINE: CH11

(ix) FEATURE:
- (A) NAME/KEY: CDS
- (B) LOCATION:1..477

(ix) FEATURE:
- (A) NAME/KEY: mat_peptide
- (B) LOCATION:67..477

(ix) FEATURE:

(A) NAME/KEY: sig_peptide
(B) LOCATION:1..66

(xi) SEQUENCE DESCRIPTION: SEQ ID NO: 11:

```
ATG AAG ACC CAC CTG CTT CTC TGG GGA GTC CTC GCC ATT TTT GTT AAG        48
Met Lys Thr His Leu Leu Leu Trp Gly Val Leu Ala Ile Phe Val Lys
-22     -20                 -15                 -10

GCT GTC CTT GTA ACA GGT GAC GAC GAA GCG ACC ATT CTT GCT GAC AAC        96
Ala Val Leu Val Thr Gly Asp Asp Glu Ala Thr Ile Leu Ala Asp Asn
     -5                  1               5                  10

AAA TGC ATG TGT ACC CGA GTT ACC TCT AGG ATC ATC CCT TCC ACC GAG       144
Lys Cys Met Cys Thr Arg Val Thr Ser Arg Ile Ile Pro Ser Thr Glu
                 15                  20                  25

GAT CCT AAT GAG GAC ATT GTG GAG AGA AAT ATC CGA ATT GTT GTC CCT       192
Asp Pro Asn Glu Asp Ile Val Glu Arg Asn Ile Arg Ile Val Val Pro
             30                  35                  40

TTG AAC AAC AGG GAG AAT ATC TCT GAT CCC ACC TCC CCA CTG AGA AGG       240
Leu Asn Asn Arg Glu Asn Ile Ser Asp Pro Thr Ser Pro Leu Arg Arg
         45                  50                  55

AAC TTT GTA TAC CAT TTG TCA GAC GTC TGT AAG AAA TGC GAT CCT GTG       288
Asn Phe Val Tyr His Leu Ser Asp Val Cys Lys Lys Cys Asp Pro Val
     60                  65                  70

GAA GTG GAG CTG GAA GAT CAG GTT GTT ACT GCC ACC CAG AGC AAC ATC       336
Glu Val Glu Leu Glu Asp Gln Val Val Thr Ala Thr Gln Ser Asn Ile
 75                  80                  85                  90

TGC AAT GAG GAC GAT GGT GTT CCT GAG ACC TGC TAC ATG TAT GAC AGA       384
Cys Asn Glu Asp Asp Gly Val Pro Glu Thr Cys Tyr Met Tyr Asp Arg
                 95                 100                 105

AAC AAG TGC TAT ACC ACT ATG GTC CCA CTT AGG TAT CAT GGT GAG ACC       432
Asn Lys Cys Tyr Thr Thr Met Val Pro Leu Arg Tyr His Gly Glu Thr
            110                 115                 120

AAA ATG GTG CAA GCA GCC TTG ACC CCC GAT TCT TGC TAC CCT GAC           477
Lys Met Val Gln Ala Ala Leu Thr Pro Asp Ser Cys Tyr Pro Asp
        125                 130                 135

TAG                                                                   480
```

(2) INFORMATION FOR SEQ ID NO: 12:

(i) SEQUENCE CHARACTERISTICS:
(A) LENGTH: 159 amino acids
(B) TYPE: amino acid
(D) TOPOLOGY: linear

(ii) MOLECULE TYPE: protein
(xi) SEQUENCE DESCRIPTION: SEQ ID NO: 12:

```
Met Lys Thr His Leu Leu Leu Trp Gly Val Leu Ala Ile Phe Val Lys
-22     -20                 -15                 -10

Ala Val Leu Val Thr Gly Asp Asp Glu Ala Thr Ile Leu Ala Asp Asn
    -5                  1               5                   10

Lys Cys Met Cys Thr Arg Val Thr Ser Arg Ile Ile Pro Ser Thr Glu
                15                  20                  25

Asp Pro Asn Glu Asp Ile Val Glu Arg Asn Ile Arg Ile Val Val Pro
            30                  35                  40

Leu Asn Asn Arg Glu Asn Ile Ser Asp Pro Thr Ser Pro Leu Arg Arg
        45                  50                  55

Asn Phe Val Tyr His Leu Ser Asp Val Cys Lys Lys Cys Asp Pro Val
    60                  65                  70

Glu Val Glu Leu Glu Asp Gln Val Val Thr Ala Thr Gln Ser Asn Ile
75                  80                  85                  90

Cys Asn Glu Asp Asp Gly Val Pro Glu Thr Cys Tyr Met Tyr Asp Arg
                95                  100                 105

Asn Lys Cys Tyr Thr Thr Met Val Pro Leu Arg Tyr His Gly Glu Thr
            110                 115                 120

Lys Met Val Gln Ala Ala Leu Thr Pro Asp Ser Cys Tyr Pro Asp
        125                 130                 135
```

(2) INFORMATION FOR SEQ ID NO: 13:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: protein

(v) FRAGMENT TYPE: N-terminal

(xi) SEQUENCE DESCRIPTION: SEQ ID NO: 13:

```
Glu Val Gln Leu Gln Gln Ser Gly Pro Glu Leu Val Lys Pro Gly
1               5                   10                  15
```

(2) INFORMATION FOR SEQ ID NO: 14:

(i) SEQUENCE CHARACTERISTICS:
(A) LENGTH: 21 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 14:

```
Asp Val Val Met Thr Gln Ser Pro Leu Ser Leu Pro Val Ser Leu Gly
1               5                   10                  15

Asp Gln Ala Ser Ile
            20
```

(2) INFORMATION FOR SEQ ID NO: 15:

(i) SEQUENCE CHARACTERISTICS:
(A) LENGTH: 391 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Mus musculus
(G) CELL TYPE: Hybridoma
(H) CELL LINE: CH11

(ix) FEATURE:
(A) NAME/KEY: CDS
(B) LOCATION:2..391

(ix) FEATURE:
(A) NAME/KEY: mat_peptide
(B) LOCATION:32..391

(ix) FEATURE:
(A) NAME/KEY: sig_peptide
(B) LOCATION:2..31

(xi) SEQUENCE DESCRIPTION: SEQ ID NO: 15:

```
C CTC CTG TCA GGA ACT GCA GGC GTC CAC TCT GAG GTC CAG CTT CAG          46
  Leu Leu Ser Gly Thr Ala Gly Val His Ser Glu Val Gln Leu Gln
  -10                 -5                   1               5

CAG TCA GGA CCT GAG CTG GTG AAA CCT GGG GCC TCA GTG AAG ATA TCC        94
Gln Ser Gly Pro Glu Leu Val Lys Pro Gly Ala Ser Val Lys Ile Ser
                 10                  15                  20
```

```
TGC AAG GCT TCT GGA TAC ACA TTC ACT GAC TAC AAC ATG CAC TGG GTG         142
Cys Lys Ala Ser Gly Tyr Thr Phe Thr Asp Tyr Asn Met His Trp Val
             25                  30                  35

AAG CAG AGC CAT GGA AAG AGC CTT GAG TGG ATT GGA TAT ATT TAT CCT         190
Lys Gln Ser His Gly Lys Ser Leu Glu Trp Ile Gly Tyr Ile Tyr Pro
         40                  45                  50

TAC AAT GGT GGT ACT GGC TAC AAC CAG AAG TTC AAG AGC AAG GCC ACA         238
Tyr Asn Gly Gly Thr Gly Tyr Asn Gln Lys Phe Lys Ser Lys Ala Thr
     55                  60                  65

TTG ACT GTT GAC AAT TCC TCC AGC ACA GCC TAC ATG GAG CTC CGC AGC         286
Leu Thr Val Asp Asn Ser Ser Ser Thr Ala Tyr Met Glu Leu Arg Ser
 70                  75                  80                  85

CTG ACA TCT GAG GAC TCT GCA GTC TAT TAC TGT GCA AGA AGT TAC TAT         334
Leu Thr Ser Glu Asp Ser Ala Val Tyr Tyr Cys Ala Arg Ser Tyr Tyr
                 90                  95                 100

GCT ATG GAC TAC TGG GGT CAA GGA ACC TCA GTC ACC GTC TCC TCA GAG         382
Ala Met Asp Tyr Trp Gly Gln Gly Thr Ser Val Thr Val Ser Ser Glu
            105                 110                 115

AGT CAG TCC                                                             391
Ser Gln Ser
        120
```

(2) INFORMATION FOR SEQ ID NO: 16:

(i) SEQUENCE CHARACTERISTICS:
- (A) LENGTH: 388 base pairs
- (B) TYPE: nucleic acid
- (C) STRANDEDNESS: double
- (D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
- (A) ORGANISM: Mus musculus
- (G) CELL TYPE: Hybridoma
- (H) CELL LINE: CH11

(ix) FEATURE:
- (A) NAME/KEY: CDS
- (B) LOCATION:2..388

(ix) FEATURE:
- (A) NAME/KEY: mat_peptide
- (B) LOCATION:29..388

(ix) FEATURE:
- (A) NAME/KEY: sig_peptide
- (B) LOCATION:2..28

(xi) SEQUENCE DESCRIPTION: SEQ ID NO: 16:

```
G ATG TTC TGG ATT CCT GCT TCC AGC AGT GAT GTT GTG ATG ACC CAA         46
  Met Phe Trp Ile Pro Ala Ser Ser Ser Asp Val Val Met Thr Gln
   -9              -5                   1               5

AGT CCA CTC TCC CTG CCT GTC AGT CTT GGA GAT CAA GCC TCC ATC TCT       94
Ser Pro Leu Ser Leu Pro Val Ser Leu Gly Asp Gln Ala Ser Ile Ser
            10                  15                  20

TGC AGA TCT AGT AAG AGC CTT GTA CAC AGT AAT GGA AAC ACC TAT TTA      142
Cys Arg Ser Ser Lys Ser Leu Val His Ser Asn Gly Asn Thr Tyr Leu
        25                  30                  35

CAT TGG TAC CTG CAG AAG CCA GGC CAG TCT CCA AAG CTC CTG ATC TAC      190
His Trp Tyr Leu Gln Lys Pro Gly Gln Ser Pro Lys Leu Leu Ile Tyr
    40                  45                  50

AAA GTT TCC AAC CGA TTT TCT GGG GTC CCA GAC AGG TTC AGT GGC AGT      238
Lys Val Ser Asn Arg Phe Ser Gly Val Pro Asp Arg Phe Ser Gly Ser
 55                  60                  65                  70

GGA TCA GGG ACA GAT TTC ACA CTC AAG ATC AGC AGA GTG GAG GCT GAG      286
Gly Ser Gly Thr Asp Phe Thr Leu Lys Ile Ser Arg Val Glu Ala Glu
                75                  80                  85

GAT CTG GGA GTT TAT TTC TGC TCT CAA AGT ACA CAT GTT CCT CCG GCG      334
Asp Leu Gly Val Tyr Phe Cys Ser Gln Ser Thr His Val Pro Pro Ala
            90                  95                 100

TTC GGT GGA GGC ACC AAG CTG GAA ATC AAA CGG GCT GAT GCT GCA CCA      382
Phe Gly Gly Gly Thr Lys Leu Glu Ile Lys Arg Ala Asp Ala Ala Pro
        105                 110                 115

ACT GTA                                                              388
Thr Val
    120
```

(2) INFORMATION FOR SEQ ID NO: 17:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 17:

CTAAGGGAAT TCCGCCTCTC CTCAGACACT GAA 33

(2) INFORMATION FOR SEQ ID NO: 18:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 18:

TTTTACTCTA GAGACCCAAG GCCTGCCTGG TTGA 34

(2) INFORMATION FOR SEQ ID NO: 19:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 19:

AAATAGGAAT TCCAGTCTCC TCAGGCTGTC TCC 33

(2) INFORMATION FOR SEQ ID NO: 20:

(i) SEQUENCE CHARACTERISTICS:
(A) LENGTH: 32 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 20:

ATGATCTCTA GAGTGGTGGC ATCTCAGGAC CT 32

(2) INFORMATION FOR SEQ ID NO: 21:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 21:

TTGCGGAATT CCTCACCTGT CCTGGGGTTA TT 32

(2) INFORMATION FOR SEQ ID NO: 22:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 22:

ATTGCCTCTA GAGCCTCTAA GGACAACGAG CT 32

(2) INFORMATION FOR SEQ ID NO: 23:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 23:

TGGGGCCTCA GTGAAGATAT 20

(2) INFORMATION FOR SEQ ID NO: 24:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 24:

CAATGGTGGT ACTGGCTACA 20

(2) INFORMATION FOR SEQ ID NO: 25:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 25:

TGACATCTGA GGACTCTGCA 20

(2) INFORMATION FOR SEQ ID NO: 26:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 26:

TCCTCAGAGA GTCAGTCCTT 20

(2) INFORMATION FOR SEQ ID NO: 27:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 27:

TCCTTCACCT GGAACTACCA 20

(2) INFORMATION FOR SEQ ID NO: 28:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 28:

TCCCAAGAGC ATCCTTGAAG 20

(2) INFORMATION FOR SEQ ID NO: 29:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 29:

AGATCTGCAT GTGCCCATTC 20

(2) INFORMATION FOR SEQ ID NO: 30:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 30:

TCTAAACTCA TCTGCGAGGC 20

(2) INFORMATION FOR SEQ ID NO: 31:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 31:

GGTGACCATC GAGAACAAAG 20

(2) INFORMATION FOR SEQ ID NO: 32:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 32:

AGGGGTCTCA CCTTCTTGAA 20

(2) INFORMATION FOR SEQ ID NO: 33:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 33:

TCCTTTGCCG ACATCTTCCT 20

(2) INFORMATION FOR SEQ ID NO: 34:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 34:

GTGTGTACTG TGACTCACAG 20

(2) INFORMATION FOR SEQ ID NO: 35:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 35:

AACTGAACCT GAGGGAGTCA 20

(2) INFORMATION FOR SEQ ID NO: 36:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 36:

AACTCTTGCC CCAAGAGAAG 20

(2) INFORMATION FOR SEQ ID NO: 37:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 37:

ATCCTGACTG TGACAGAGGA 20

(2) INFORMATION FOR SEQ ID NO: 38:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 38:

ACAAGTCCAC TGGTAAACCC 20

(2) INFORMATION FOR SEQ ID NO: 39:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 39:

AGGATATCTT CACTGAGGCC 20

(2) INFORMATION FOR SEQ ID NO: 40:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 40:

ATCCACTCAA GGCTCTTTCC 20

(2) INFORMATION FOR SEQ ID NO: 41:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 41:

ACTGCAGAGT CCTCAGATGT 20

(2) INFORMATION FOR SEQ ID NO: 42:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 42:

AGACGGTGAC TGAGGTTCTT 20

(2) INFORMATION FOR SEQ ID NO: 43:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 43:

CAGGTGAAGG AAATGGTGCT 20

(2) INFORMATION FOR SEQ ID NO: 44:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 44:

ATGCTCTTGG GAGACAGCAA 20

(2) INFORMATION FOR SEQ ID NO: 45:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 45:

CTCTGTTTTT GCCTCCGTAG 20

(2) INFORMATION FOR SEQ ID NO: 46:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 46:

TGGCCTCGCA GATGAGTTTA 20

(2) INFORMATION FOR SEQ ID NO: 47:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 47:

CCTTTGTTCT CGATGGTCAC 20

(2) INFORMATION FOR SEQ ID NO: 48:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 48:

TGTGGAGGAC ACGTTCTTCA 20

(2) INFORMATION FOR SEQ ID NO: 49:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 49:

ACTTTGAGAA GCCCAGGAGA 20

(2) INFORMATION FOR SEQ ID NO: 50:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 50:

AGATCCCTGT GAGTCACAGT 20

(2) INFORMATION FOR SEQ ID NO: 51:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 51:

AGCAGGTGGA TGTTTGTGCA 20

(2) INFORMATION FOR SEQ ID NO: 52:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 52:

TGAAGCCACT GCACACTGAT 20

(2) INFORMATION FOR SEQ ID NO: 53:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 53:

AGTTCCATTC CTCCTCTGTC 20

(2) INFORMATION FOR SEQ ID NO: 54:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 54:

TGTGTCAGAC ATGATCAGGG 20

(2) INFORMATION FOR SEQ ID NO: 55:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 55:

TGAAGTTGCC TGTTAGGCTG 20

(2) INFORMATION FOR SEQ ID NO: 56:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 56:

CTTGGAGATC AAGCCTCCAT 20

(2) INFORMATION FOR SEQ ID NO: 57:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 57:

GCTGAGGATC TGGGAGTTTA 20

(2) INFORMATION FOR SEQ ID NO: 58:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 58:

GATGCTGCAC CAACTGTATC 20

(2) INFORMATION FOR SEQ ID NO: 59:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 59:

CGACAAAATG GCGTCCTGAA 20

(2) INFORMATION FOR SEQ ID NO: 60:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 60:

ACGTTGACCA AGGACGAGTA 20

(2) INFORMATION FOR SEQ ID NO: 61:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 61:

ATCTGCAAGA GATGGAGGCT 20

(2) INFORMATION FOR SEQ ID NO: 62:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 62:

ACCCCAGAAA ATCGGTTGGA 20

(2) INFORMATION FOR SEQ ID NO: 63:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 63:

CCGGAGGAAC ATGTGTACTT 20

(2) INFORMATION FOR SEQ ID NO: 64:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 64:

TCGTTCATAC TCGTCCTTGG 20

(2) INFORMATION FOR SEQ ID NO: 65:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 65:

CATCTCAGGA CCTTTGTCTC 20

(2) INFORMATION FOR SEQ ID NO: 66:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 66:

CACCTGTCCT GGGGTTATTT 20

(2) INFORMATION FOR SEQ ID NO: 67:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 67:

AGACAAGATG AAGACCCACC 20

(2) INFORMATION FOR SEQ ID NO: 68:

(i) SEQUENCE CHARACTERISTICS:
(A) LENGTH: 20 base pairs

(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 68:

AAGCGACCAT TCTTGCTGAC 20

(2) INFORMATION FOR SEQ ID NO: 69:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 69:

ATATCTCTGA TCCCACCTCC 20

(2) INFORMATION FOR SEQ ID NO: 70:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 70:

GAAATGCGAT CCTGTGGAAG 20

(2) INFORMATION FOR SEQ ID NO: 71:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 71:

CTATACCACT ATGGTCCCAC 20

(2) INFORMATION FOR SEQ ID NO: 72:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 72:

AGAAGCAGGT GGGTCTTCAT 20

(2) INFORMATION FOR SEQ ID NO: 73:

(i) SEQUENCE CHARACTERISTICS:
(A) LENGTH: 20 base pairs
(B) TYPE: nucleic acid

(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 73:

TAGAGGTAAC TCGGGTACAC 20

(2) INFORMATION FOR SEQ ID NO: 74:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 74:

AAGTTCCTTC TCAGTGGGGA 20

(2) INFORMATION FOR SEQ ID NO: 75:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 75:

GGTGGCAGTA ACAACCTGAT 20

(2) INFORMATION FOR SEQ ID NO: 76:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 76:

CATGATACCT AAGTGGGACC 20

(2) INFORMATION FOR SEQ ID NO: 77:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 77:

GGGGAATTCC AGTACGGAGT TGGGGAAGCT CTTT 34

(2) INFORMATION FOR SEQ ID NO: 78:

(i) SEQUENCE CHARACTERISTICS:
(A) LENGTH: 35 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 78:

GTTTCTTCTG CCTCTGTCAC CAAGTTAGAT CTGGA 35

(2) INFORMATION FOR SEQ ID NO: 79:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 79:

TCCAGATCTA ACTTGGTGAC AGAGGCAGAA GAAAC 35

(2) INFORMATION FOR SEQ ID NO: 80:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 80:

CCCTCTAGAC GCGTCACGTG GGCATCAC 28